(19) **Europäisches Patentamt / European Patent Office / Office européen des brevets**

(11) **EP 1 983 920 B1**

(12) **EUROPEAN PATENT SPECIFICATION**

(45) Date of publication and mention
of the grant of the patent:
**03.08.2016 Bulletin 2016/31**

(21) Application number: **07811790.0**

(22) Date of filing: **01.02.2007**

(51) Int Cl.:
***A61B 5/00*** (2006.01)

(86) International application number:
**PCT/US2007/061463**

(87) International publication number:
**WO 2007/143236 (13.12.2007 Gazette 2007/50)**

(54) **METHODS AND SYSTEMS FOR MONITORING AND OBTAINING INFORMATION OF AT LEAST ONE PORTION OF A SAMPLE USING CONFORMAL LASER THERAPY PROCEDURES, AND PROVIDING ELECTROMAGNETIC RADIATION THERETO**

VERFAHREN UND SYSTEME ZUR INFORMATIONSÜBERWACHUNG UND ERFASSUNG VON MINDESTENS EINEM TEIL EINER PROBE MITTELS KONFORMER LASERTHERAPIEVERFAHREN SOWIE BEREITSTELLUNG VON ELEKTROMAGNETISCHER STRAHLUNG DAFÜR

PROCÉDÉS ET SYSTÈMES DE CONTRÔLE ET D'OBTENTION D'INFORMATIONS SUR AU MOINS UNE PORTION D'UN ÉCHANTILLON UTILISANT DES PROCÉDURES DE THÉRAPIE LASER CONFORMES, ET PROCURANT UN RAYONNEMENT ÉLECTROMAGNÉTIQUE CORRESPONDANT

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.02.2006 US 764622 P**
**01.06.2006 US 810445 P**

(43) Date of publication of application:
**29.10.2008 Bulletin 2008/44**

(73) Proprietor: **The General Hospital Corporation Boston, MA 02114 (US)**

(72) Inventors:
• **TEARNEY, Guillermo J.**
**Cambridge, MA 02139 (US)**

• **SHISHKOV, Millen**
**Watertown, MA 02472 (US)**
• **BOUMA, Brett E.**
**Quincy, MA 02171 (US)**
• **VAKOC, Benjamin J.**
**Massachusetts 02474 (US)**

(74) Representative: **Quinterno, Giuseppe et al**
**Jacobacci & Partners S.p.A.**
**Corso Emilia 8**
**10152 Torino (IT)**

(56) References cited:
**EP-A- 0 933 096    WO-A-96/28212**

**Description**

**FIELD OF THE INVENTION**

The present invention relates to systems and ex-vivo methods for obtaining information associated with at least one characteristic of a biological sample.

**BACKGROUND OF THE INVENTION**

A use of lasers for ablating or thermally destroying diseased tissue is known and at time preferred, primarily due to the potential for precise local effect with minimal collateral damage.

Such devices are disclosed in e.g. WO96/28212 and EP 0 933 096 A2.

In practice, however, laser therapy has been less than perfect for use in certain clinical applications, such as the treatment of early epithelial cancers and their precursors. One of the problems with laser therapy for these applications has been the inability to accurately control and guide the treatment depth, resulting in either disease recurrence due to incomplete therapy or complications associated with overly aggressive treatment.

*Epithelial cancer: diagnosis and treatment*

Methods and techniques for identifying and treating cancer at an early stage have been widely pursued as offering the potential to dramatically decrease the morbidity and mortality associated with metastasis. Since epithelial cancers and precursor lesions are frequently focal and can be distributed heterogeneously across a wide field, a sensitive diagnosis is extremely demanding. A diagnosis should be rendered on the size scale of a single cell in a field comprising possibly more than a billion cells.

Epithelial cancer also presents challenges for therapy. Since they are superficial, access to epithelial lesions can frequently be obtained through the use of minimally invasive catheters or endoscope. The therapeutic challenge, however, is in comprehensively killing, resecting or ablating the entire lesion without damage to underlying or adjacent tissues. This is particularly challenging since the depth of disease and even the thickness of normal epithelial layers can vary substantially. Additionally, epithelial tissues are highly compliant and therapeutic instrumentation can result in significant compression. As a result, therapies designed to affect tissue to a fixed depth risk either under-treatment resulting in recurrence, or over-treatment that can lead to significant complications.

*Barrett's Esophagus*

The importance of Barrett's esophagus (BE) is based primarily on the prevalence of this disease, the rapid increase in its incidence, and the dismal prognosis for patients with high-grade dysplasia and adenocarcinoma. as described in publication 1 identified below. The current consensus (as described in publications 2 and 3 identified below) holds that comprehensive destruction of BE in a controlled fashion, along with anti-reflux treatment, results in squamous regrowth and that continued reflux control prevents the return of BE. The challenge is in achieving comprehensive removal of the pathologic mucosa, while preserving the underlying tissues of the esophageal wall. Treatment that is incomplete can result in a squamous overgrowth that masks underlying pathology. Overly aggressive therapy can result in stricture or perforation of the esophageal wall. Provided below is the information relating to screening and therapy of BE.

*Screening*

Several approaches for esophageal screening in the management of BE have been investigated. Brush cytology (as described in publications 4 and 5 identified below) and the use of biological markers, such as the deletion and/or mutation of the 17p (p53) gene, (as described in publications 6 and 7 identified below) can be used independently of endoscopy but cannot provide spatial mapping of disease. High magnification video endoscopy (as described in publication 8 identified below), fluorescence spectroscopy (as described in publications 9 identified below), and light-scattering spectroscopy (as described in publications 10 identified below) each show promise for point diagnoses, but provide insufficient information regarding subsurface microstructure and have not been demonstrated for wide-field screening. High-resolution endoscopic ultrasound and chromoendoscopy (as described in publications 11 and 12 identified below, respectively) can both be applied to a wide field, but have suffered from low sensitivity and specificity.

Optical coherence tomography (OCT) system, methods and techniques (as described in publications 13 and 14 identified below) has been developed. Certain accurate OCT diagnostic criteria have been developed for specialized intestinal metaplasia, dysplasia and adenocarcinoma, as described in International Patent Application PCT/US2004/029148, filed September 8, 2004, U.S. Patent Application No. 10/501,276, filed July 9, 2004, and publications 15-17 identified below. For example, advances in OCT technology have occurred demonstrating that the acquisition of an OCT signal in the wavelength domain (as opposed to the time domain) can provide orders of magnitude improvement in imaging speed while maintaining excellent image quality, as described in publications 18-20 identified below. One such exemplary second-generation imaging technology has been developed, e.g., optical frequency domain imaging (OFDI), as described in U.S. Patent Application No. 11/266,779, filed November 2, 2005 and publication 21 identified below. With OFDI methods, techniques and systems, high-resolution ranging can be conducted in a tissue by detecting spectrally-resolved interference between the tissue sample and a reference while the source wavelength is tuned. (See, e.g., publication 22 identified below). Currently, OFDI methods, techniques and systems may be capable of capturing (e.g., 10 $\mu$m) 3 voxels at rates of approximately 40 million per second and the imaging speeds may likely be more than double in the near future, as provided in publication 23 identified below. Additionally, phase-sensitive OFDI methods, techniques and systems has been used for imaging flow, as provided in publication 24 identified below.

*Controllable Therapy*

Certain endoluminal approaches have been evaluated for the treatment of SIM (with and without dysplasia), including photodynamic therapy (PDT) (as provided in reference 25 identified below), laser (532 nm and 1064 nm) (as provided in reference 26 identified below), multipolar electrocoagulation (as provided in reference 27 identified below), argon plasma coagulation (as provided in reference 28 identified below), endoscopic mucosal resection (as provided in reference 29 identified below), radiofrequency ablation (as provided in reference 30 identified below) and cryoablation (as provided in reference 31 identified below) using liquid nitrogen. Although each of these techniques appear to be successful, most studies describe nonuniform therapy that can potentially result in persistent SIM or excessively deep ablation, resulting in stricture or perforation. In a study of over 100 patients, PDT may result in a stricture rate of 30% for single treatments and 50% for more than one treatment (as provided in reference 32 identified below). An exemplary reason for failure is not entirely clear but possible contributing causes include the operator-dependent nature of many of these hand-held, hand-aimed devices, the large surface area that requires treatment and the inherent preference for a physician-determined visual end point for the treatment (as provided in references 3 and 30 identified below). Additionally, a high variability may exist in the thickness of mucosal layers within and between patients and have directly observed significant compression of the soft tissues of the esophagus. The prior therapeutic approaches, however, do not account for the variability of layer thickness or compressibility of the esophageal wall.

Accordingly, there is a need to overcome the deficiencies described herein above.

## OBJECTS AND SUMMARY OF THE INVENTION

To address and/or overcome the above-described problems and/or deficiencies as well as other deficiencies, exemplary embodiments of methods and systems can be provided for monitoring at least one portion of a sample, providing electromagnetic radiation thereto and obtaining information associated with at least one characteristic of the sample.

Such deficiencies can be addressed using the exemplary embodiments of the present invention.

The invention is defined in the claims.

In one exemplary embodiment of the present invention, a method and system are provided for obtaining information associated with at least one portion of a sample. For example, a temperature change can be caused in the portion of the sample. At least one first electro-magnetic radiation can be forwarded to a section near or in the portion of the sample. A deformation of the section can be identified at a plurality of depths as a function of (i) a phase of at least one second electro-magnetic radiation provided from the section, and/or (ii) a rate of change of the phase and/or an amplitude of the second electro-magnetic radiation.

It is possible to generate an interferometric signal associated with the second electro-magnetic radiation, and determine the phase of the second electro-magnetic radiation using the interferometric signal. The interferometric signal can be measured as a function of a wavelength of the second electro-magnetic radiation. The first electro-magnetic radiation can have a wavelength that varies over time. The temperature change may be caused using a laser arrangement. A border can be defined between the at least one changed portion and an unchanged portion of the sample as a function of the information associated with the deformation. The sample can be a biological structure, and the changed portion may be denatured, damaged and/or destroyed. In addition, an interferometric signal associated with the second electro-magnetic radiation can be generated, and the amplitude of the second electro-magnetic radiation can be determined using the interferometric signal. The interferometric signal can be measured as a function of a wavelength of the second electro-magnetic radiation.

In another exemplary embodiment , a method and system are provided for controlling a temperature distribution in a sample. For example, an electro-magnetic radiation can be provided to the section in the sample at a particular wavelength. The temperature distribution can be controlled by modifying the particular wavelength of the electro-magnetic radiation when the electro-magnetic radiation is provided to the section.

In particular, the modification to the particular wavelength can change a distribution of damage in at least one portion of the sample. The temperature distribution can be further controlled by modifying a power of the electro-magnetic radiation. The particular wavelength may be modified to be in a range of approximately (i) about 1.35 $\mu$m to 1.5 $\mu$m and/or (ii) about 1.7 $\mu$m to 2.2 $\mu$m. The temperature distribution can be substantially due to an absorption of the electro-magnetic radiation by water. The electro-magnetic radiation can be provided by a thulium laser amplifier arrangement and/or an erbium laser amplifier arrangement. A rate at which the particular wavelength is modified can be greater that about 10 nm per second. The particular wavelength may be modified in a non-random manner.

These and other objects, features and advantages of the present invention will become apparent upon reading the following detailed description of embodiments of the invention, when taken in conjunction with the appended claims.

## BRIEF DESCRIPTION OF THE DRAWINGS

Further objects, features and advantages of the present invention will become apparent from the following detailed description taken in conjunction with the accompanying figures showing illustrative embodiments of the present invention, in which:

Fig. 1A is a schematic diagram of an OFDI balloon catheter in accordance with an exemplary embodiment of the present invention;

Fig. 1B is a photograph of the OFDI balloon catheter shown in Fig. 1A;

Fig. 2A is an exemplary image of a perspective view of a swine esophagus obtained using the OFDI balloon catheter in accordance with an exemplary embodiment of the present invention;

Fig. 2B is an exemplary image of a top view of the swine esophagus of Fig. 2A;

Fig. 2C is an exemplary image of a side view of a wall of the swine esophagus of Fig. 2A;

Fig. 3 is an exemplary OFDI image acquired in a human subject using a BE technique;

Fig. 4 is a schematic diagram of exemplary arrangement and usage thereof for treating and monitoring tissue in accordance with an exemplary embodiment of the present invention;

Fig. 5 is a set of multiple exemplary m-mode OFDI phase images obtained using the exemplary arrangement of Fig. 4 together with a corresponding histology;

Figs. 6A-6D are exemplary images associated with the OFDI data acquired for a translating sample in accordance with the exemplary embodiment of the present invention;

Fig. 7A is an exemplary pre-laser treatment OFDI image obtained using the exemplary embodiment of the present invention;

Fig. 7B is an exemplary pre-laser treatment birefringence image obtained using the exemplary embodiment of the present invention;

Fig. 7C is an exemplary post-laser treatment OFDI image obtained using the exemplary embodiment of the present invention;

Fig. 7D is an exemplary post-laser treatment birefringence image obtained using the exemplary embodiment of the present invention;

Fig. 8 is an image of an exemplary vascular map extracted from a comprehensive dataset obtained from porcine esophagus in-vivo which can be obtained using the exemplary embodiment of the present invention;

Fig. 9 is an exemplary in-vivo Doppler flow image of a porcine esophagus obtained using the exemplary embodiment of the present invention;

Fig. 10 is an plot of water absorption coefficient and corresponding penetration depth as a function of wavelength obtained using the exemplary embodiment of the present invention;

Fig. 11 is a schematic diagram of a two beam catheter probe in accordance with another exemplary embodiment of the present invention;

Fig. 12 is schematic side and front illustrations of a three beam catheter probe in accordance with yet another exemplary embodiment of the present invention;

Fig. 13 is a perspective view of a watch-spring multichannel optical rotary junction in accordance with an exemplary embodiment of the present invention;

Fig. 14 is a conceptual rendering of an image which can provide feedback to a user obtained using an exemplary embodiment of the present invention;

Fig. 15 is a block diagram of a sample arm of an OFDI system incorporating an optical switch in accordance with a further exemplary embodiment of the present invention;

Fig. 16 is a block diagram of the sample arm of the OFDI system incorporating an optical splitter in accordance with a still further exemplary embodiment of the present invention;

Fig. 17 is a block diagram of the sample arm of the OFDI system incorporating a single wavelength-division multiplexer in accordance with yet further exemplary embodiment of the present invention;

Fig. 18 is a block diagram of the sample arm of the OFDI system incorporating a cladding mode coupler and a dual-clad fiber in accordance with still another exemplary embodiment of the present invention;

Fig. 19 is a block diagram of a three-port rotary coupler and catheter in accordance with an exemplary embodiment of the present invention;

Fig. 20 is a block diagram of a single-fiber rotary coupler with subsequent demultiplexing of the therapy light and capable of splitting of imaging light in accordance with another exemplary embodiment of the present invention;

Fig. 21 is a schematic diagram and usage of a two-beam in-line catheter probe in accordance with an exemplary embodiment of the present invention;

Fig. 22 are front and side illustrations of a three-beam catheter probe and balloon catheter in accordance with an exemplary embodiment of the present invention;

Fig. 23 is a side view of a micro-motor-based arrangement capable of generating a slowly rotatable therapy beam and fast scanning imaging beam in accordance with an exemplary embodiment of the present invention;

Fig. 24 is a block diagram of a therapy source incorporating a low power tunable source followed by a broadband booster amplifier in accordance with an exemplary embodiment of the present invention;

Fig. 25 is a block diagram of a therapy source incorporating multiple laser diodes (LDs) at difference wavelengths and polarizations in accordance with another exemplary embodiment of the present invention;

Fig. 26 is an illustration of a wavelength-tunable therapy source incorporating a laser diode bar and results generated thereby in accordance with an exemplary embodiment of the present invention;

Fig. 27 is a side view of another exemplary embodiment of a system which includes a galvanometric scanner which can allow the OFDI beam to be repetitively scanned across the surface of the tissue, and usage thereof;

Fig. 28 is a schematic diagram of a further exemplary embodiment of the OFDI system according to the present invention which can be used to detect both the imaging and monitoring signals via acousto-optic frequency shifters;

Fig. 29A is a flow diagram of an exemplary embodiment of a method for obtaining information associated with at least one portion of a sample;

Fig. 29B is a flow diagram of another exemplary embodiment of the method for controlling a temperature distribution in the sample; and

Fig. 29C is a flow diagram of yet another exemplary embodiment of the method for applying a laser radiation to at least one portion of a biological structure.

Throughout the figures, the same reference numerals and characters, unless otherwise stated, are used to denote like features, elements, components or portions of the illustrated embodiments. Moreover, while the subject invention will now be described in detail with reference to the figures, it is done so in connection with the illustrative embodiments. It is intended that changes and modifications can be made to the described embodiments without departing from the true scope of the subject invention as defined by the appended claims.

## DETAILED DESCRIPTION OF EXEMPLARY EMBODIMENTS

An exemplary embodiment of the system and method according to the present invention can be based on a thermal excitation delivered by a conventional, spatially scanned laser beam. For example, in the absence of photochemical or phase transition processes, the laser energy absorbed by tissue can be substantially or entirely converted to a temperature rise, as described in publication 33 identified below. For exposure durations greater than approximately 10 ms, temperatures in excess of 60-70°C generally can lead to irreversible protein denaturation and cell death irrespective of duration, as described in publication 34 identified below. When the energy is absorbed, it can be subject to a spatial redistribution by a thermal diffusion. In 1983, as described in publication 35 identified below, an exemplary concept was described which provided that spatially confined microsurgical effects (selective photothermolysis) can be achieved by the use of laser exposures that are shorter than the characteristic thermal diffusion time of the heated volume. For a relatively large (>1mm) diameter laser beam and laser wavelengths in the vicinity of 1450 nm, this characteristic diffusion time for biological tissues may be on the order of 1 second. Under these conditions, the temperature increase can be determined by the laser power density, $P_d$, the absorption coefficient, $\mu_a$, and the duration of exposure t (as described in publications 33 and 34 identified below) as follows:

$$\Delta T(t,r,z) \approx \frac{P_d t \mu_a}{\rho c} \exp(-\mu_a z - \frac{2r^2}{W^2}) \qquad \text{Eq. 1}$$

where p is the tissue density, c the heat capacity, and r the radial distance from the center of a Gaussian laser beam of $1/e2$ radius, W. Although this approximation neglects scattering of the laser light as it propagates into the tissue, models that explicitly include scattering (as described in publication 36 identified below) indicate less than 10% deviation from Eq. 1 under the stated conditions.

Since the absorption coefficient is wavelength-dependent, Eq. 1 indicates that laser parameters $P_d$, t, and wavelength can be used to control the depth of thermal injury and to minimize collateral damage to underlying tissues. Operating in the visible portion of the spectrum is challenging since absorption is governed by a wide range of chromophores whose concentration is highly variable across different tissues and pathologic conditions. By comparison, the absorption spectrum of biological tissues near 1.45 $\mu$m may be dominated by water, and can therefore be roughly constant across a range of tissues. Additionally, by tuning over a modest wavelength range, from 1375 nm to 1430 nm for example, absorption lengths can be selected that range from over 2 mm to 300 $\mu$m. This exemplary range is well matched to the depths characteristic of epithelial lesions.

### Exemplary Monitoring

Several approaches have been investigated for monitoring laser therapy, including the analysis of the acoustic transients generated during ablation (as described in publication 37 identified below), changes in tissue reflectivity (as described in publications 38 and 39 identified below), fluorescence spectroscopy for discrimination between plaque and vessel wall (as described in publication 40 identified below), plasma spectroscopy to distinguish between bone and nerve tissue (as described in publication 41 identified below), and analysis of the cavitation bubble dynamics at the tip of a laser optical probe for controlled sclera perforation in glaucoma surgery (as described in publication 42 identified below). With the exception of the procedures that are based on a reflectivity described in publications 38 and 39, in each of such methods, the monitoring signal arose only after the zone of thermal injury has transitioned across a boundary of the specific tissue types. None could determine the depth of thermal injury or the spatial relationship of the damaged tissue to adjacent viable tissue. Certain degree of spatial resolution has been achieved by monitoring the portion of laser light that is not absorbed by the tissue. By inserting an optical fiber through a needle, this light can be collected from different perspectives surrounding the heated volume and temperature-dependent scattering changes can be measured (as described in publication 43 identified below). A more direct approach, high-resolution in situ imaging, has also been demonstrated for visualizing scattering changes and the physical removal of tissue resulting from ablative laser irradiation (as described in publication 44 identified below).

Exemplary embodiments of monitoring systems, methods and techniques according to the present invention may utilize information regarding well-known tissue responses to a thermal injury. These exemplary responses can include, but not limited to, microscopic deformation (as described in publication 33 identified below) and changes in scattering (as described in publications 36, 38 and 45 identified below), birefringence (as described in publication 46 identified below), and blood flow (as described in publication 47 identified below) that can result from laser heating and that can be observed over a range of temperatures beginning as low as 45° C. One exemplary aspect of an exemplary embodiment of the method and technique according to the present invention is that these thermal responses can be detected with high spatial resolution and presented in a cross-sectional image format along with the microscopic tissue structure.

*Exemplary Strategies for Conformal Laser Therapy*

According to an exemplary embodiment of the present invention, a system, arrangement and method can be provided that are capable of screening and delivering precisely guided laser therapy. Since the characteristic length-scales preferably usable for comprehensive screening and comprehensive therapy are likely distinct, it is possible to separately perform these objectives. For example, the screening (e.g., possibly performed as a first step) may utilize comprehensive imaging technique(s) with a resolution on the cellular size-scale. This exemplary procedure can be used to identify regions for subsequent therapy. After the performance of the screening procedure, the endoscopic probe can be directed back to the specified regions, and therapy may be performed under real-time guidance so that all disease is treated and collateral damage is minimized. This exemplary result can improve the management of patients with Barrett's esophagus by, e.g., increasing the effectiveness of therapy while decreasing the risk of complications.

Although described in conjunction with a treatment of epithelial cancers, the exemplary embodiments of the system, techniques and methods according to the present invention can be applicable to any application of laser treatment including but not limited to, for example, applications in dermatology. Some relevant epithelial cancers and precancerous lesions addressed by the exemplary embodiments of the present invention can include, but not limited to, the larynx, cervix and ovaries, bladder, oral cavity and lung. In addition, the exemplary embodiments of the present invention can be applicable to the areas of monitoring photodynamic therapy, radiofrequency ablation, and cryotherapy to provide control over depth and spatial extent of therapy.

*Exemplary Wide-field Screening*

In order to perform an effective screening procedure, it is preferable to conduct a comprehensive examination of large surface areas and the application of accurate diagnostic criteria in order to identify specific regions of pathology. Various OCT diagnostic criteria has been developed and verified for specialized intestinal metaplasia, dysplasia and adenocarcinoma, as describe in publications 15-17 identified below. For example, across 288 biopsies obtained from 121 patients, a sensitivity and specificity for diagnosing SIM (versus all other upper GI tract tissues) has been determined of about 97% and 92%, respectively, as described in publication 16 identified below. Until recently, however, the exemplary OCT technique was too slow to image large mucosal surface areas. As discussed herein below, advances have been made that may overcome this timing issue, and provide a preliminary demonstration of comprehensive esophageal imaging in vivo.

*Optical Frequency-Domain Imaging (OFDI)*

As described above, publication 21 identified below describes the development of the OFDI technique as an alternative to the use of the OCT techniques. Although the light source (as discussed in publication 22 and 23 identified below) and the detection principles of OFDI are useful, the contrast, resolution and cross-sectional image presentation are approximately equivalent or similar to those provided by OCT. One of the advantages of OFDI is that OFDI has a higher detection sensitivity, thus enabling a significant increase in the image acquisition speed, without compromising image quality. The system used for these preliminary studies was designed specifically for endoscopic imaging and provides an acquisition rate of 10,000 depth-scans (A-lines) per second, an axial resolution of 8 $\mu$m in tissue, and a ranging depth of 3.5 mm, as described in publication 24 identified below. The imaging speed of this exemplary system is limited solely by the rate at with which data can be transferred across the computer's bus and stored to a hard drive.

*Exemplary Balloon catheter*

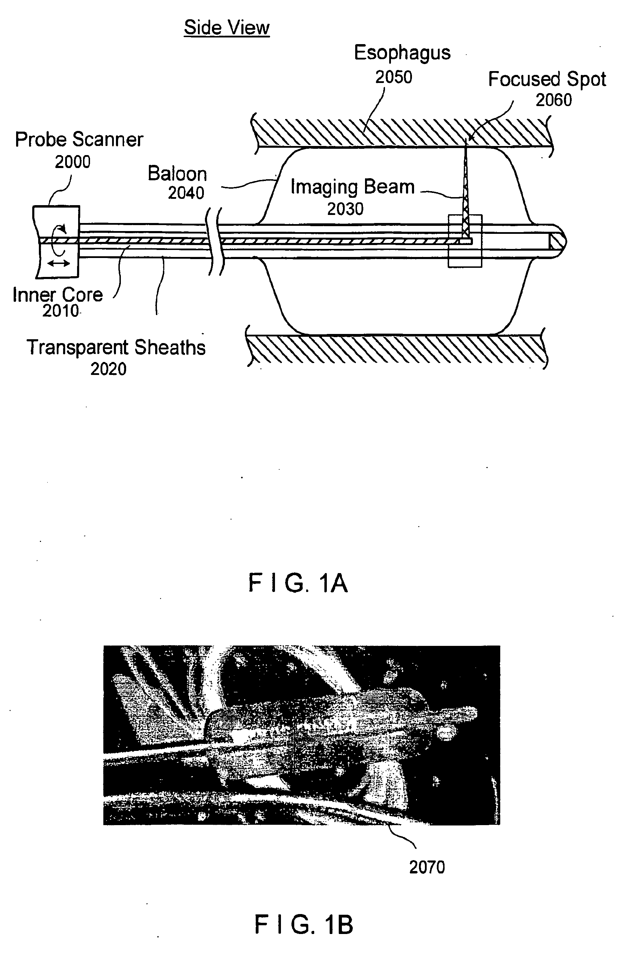

For comprehensive esophageal imaging, an exemplary embodiment of an OFDI catheter may be provided in accordance with the present invention that can be centered within the esophageal lumen using a balloon sheath shown in Figs. 1A and 1B. The exemplary catheter may include of a probe scanner 2000 which can rotate, and may pull back an inner optical core 2010. The inner core 2010 can be enclosed within a transparent sheath 2020. At the distal end of the catheter, the balloon 2040 which, when inflated, may center the imaging optics. The imaging beam 2030 can be focused onto the esophageal surface 2050. This imaging beam 2030 may be scanned to achieve comprehensive imaging. The balloon 2040 can have an inflated diameter of 1.8 cm, and may allow for a longitudinal imaging over a length of 4.5 cm without repositioning. The optical core 2010 of the catheter can include an optical fiber, a spacer for expansion of the optical beam, a gradient index lens for focusing, and a right-angle prism for directing the beam perpendicularly to the longitudinal axis of the catheter. A miniature cylindrical lens was fabricated in-house and placed on the second surface of the prism. This lens compensated for astigmatism induced by the plastic sheaths and resulted in a diffraction-limited beam (30 $\mu$m diameter) on the tissue surface. In use, the exemplary catheter may be rotated at rate of about 4 revolutions per second, allowing the acquisition of 2500 axial scans per circular cross-section. This exemplary OFDI system can record an encoder signal to precisely track the rotation and pull-back of the catheter. This information is used in reconstructing the 3-dimensional data set.

*Preliminary porcine esophageal imaging*

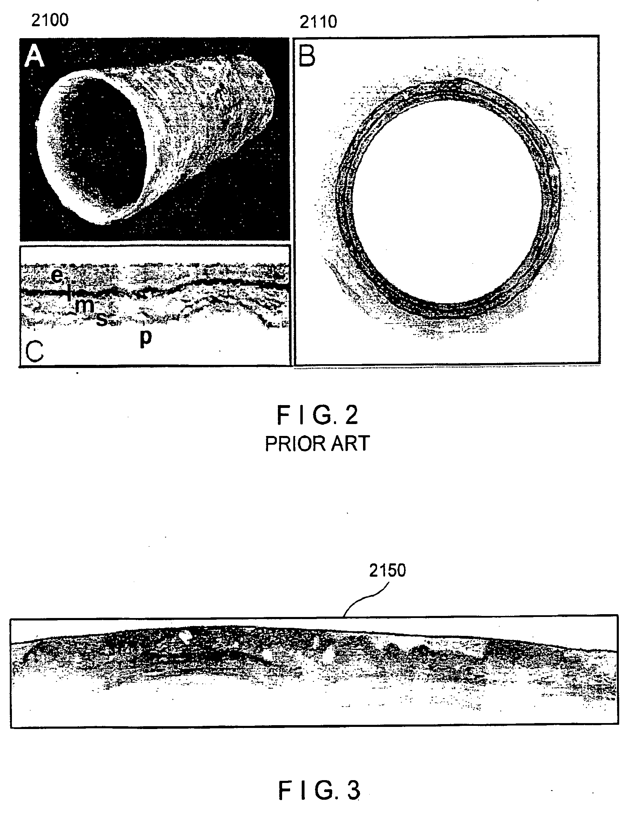

The esophageal imaging techniques can be performed in two ~50 kg swine. Although the complete 20 GB data set may likely not be represented in discrete figures, the information content is shown by Figs. 2A-2C. For example, in a perspective view of Fig. 2A, an image 2100 provides a 3D rendering of the entire imaged esophagus. In a front view of Fig. 2B, an image 2110 illustrates a single transverse cross section of the imaged esophagus. In Fig. 2C, image 2120 shows a zoomed cross sectional image of at least one portion of the esophagus. A sampling with a resolution of 10 $\mu$m x 20 $\mu$m x 30 $\mu$m (r,$\theta$,z) can yield a comprehensive microscopic data set that can be displayed volumetrically a the image 2100 of Fig. 2A for mapping and orientation, or in high-resolution cross-sectional images in which the entire esophageal wall can be visualized as the image 2110 in Fig. 2B. An expanded view of the image 2120 of Fig. 2C depicts the architectural structure of the mucosal layers.

*Preliminary human esophageal imaging*

An exemplary single rotational image 2150 is shown in Fig. 3. Hallmark features of SIM (disorganized epithelial architecture with irregular surface; presence of large epithelial glands) of a patient are shown therein. This patient had a prior diagnosis of BE, and imaging was performed prior to PDT.
These preliminary studies demonstrate that a) comprehensive OFDI microscopic imaging in vivo is feasible, b) the architectural structure of the entire esophageal wall can be visualized, and c) features important to the diagnosis of SIM in human subjects can be detected using the balloon centering probe.

*Monitoring Laser Thermal Injury*

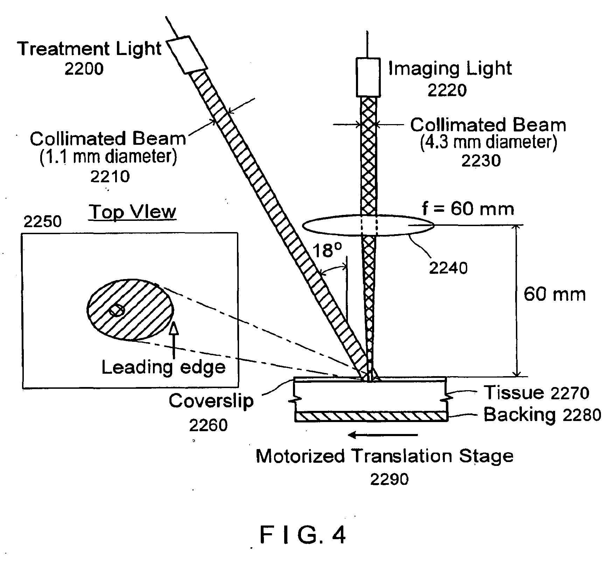

In response to heating, tissue proteins and collagen can denature, giving rise to microscopic deformation (described in publications 33 identified below), increased in scattering (described in publications 36, 38 and 45 identified below), reduced birefringence (described in publication 46 identified below), and reduced blood flow (described in publication 47 identified below). The description below provides the methods for monitoring these changes using exemplary OFDI in accordance with the exemplary embodiments of the present invention. In the exemplary demonstration of each, freshly obtained porcine esophagus samples and duodenum samples (as a proxy for SIM) were mounted with a microscope cover glass on the epithelial surface so that the approximate pressure and thermal conductivity of the balloon catheter could be simulated.
An exemplary embodiment of an apparatus for collecting OFDI signals during a laser irradiation and use thereof according to the present invention is shown in Fig. 4. For example, the treatment light is delivered through a collimator 2200. The imaging light is delivered through a second collimator 2220. The treatment beam 2210 and imaging beam 2230 overlap when reaching the tissue 2270 which is covered with a think glass cover slip 2260 and resting on a backing 2280. The tissue is translated by a motorized translation stage 2290. The imaging beam is focused by a lens 2250. The top-down image depicting beam overlap 2250 is provided. For a thermal excitation, a collimated, high-power Gaussian laser beam (e.g., diameter = 1.1 mm; wavelength = 1450 nm; power = 400 mW) can be used. The OFDI sampling beam can be focused at the tissue surface to, e.g., a $1/e^2$ intensity diameter of 23 $\mu$m and aligned so that it overlapped with the laser spot as shown in Fig. 4. During the data collection, the samples may be held at a fixed location and/or translated using a motorized stage.

### *Exemplary Microscopic Deformation*

As laser energy is deposited in tissue, the resulting temperature increase can denature proteins and collagen. These changes can be manifested by microscopic deformation that can be measured using phase-sensitive OFDI. The following data demonstrates this capability.

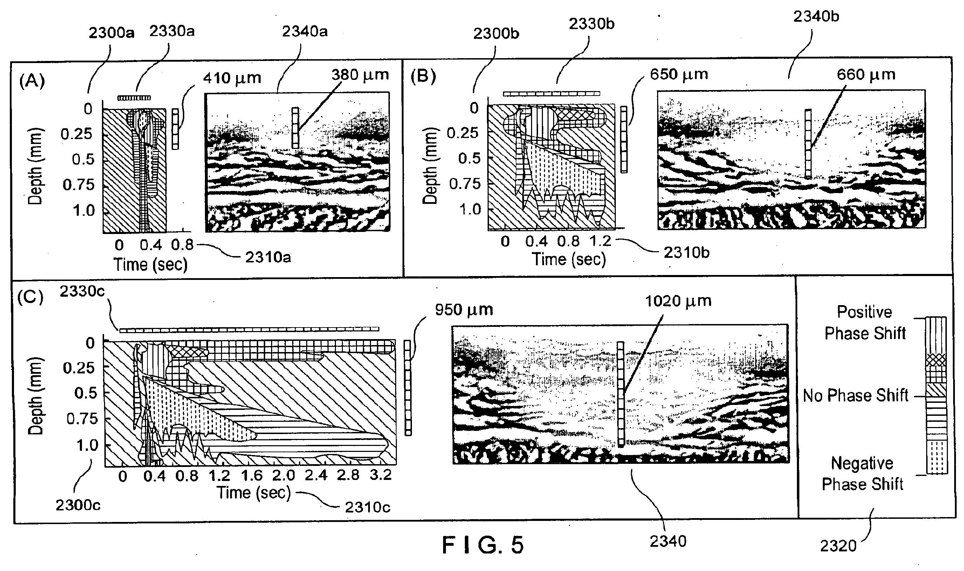

Fixed spot - For such exemplary experiment, the samples were held at a fixed location. OFDI depth-scans were acquired continuously at a rate of about 10 kHz while the 1450 nm laser was switched on, held at a constant power of 400 mW for a predetermined duration, and switched off. Representative data for three different laser exposure durations is shown in the graphs of Fig. 5 as "M-mode" images where the vertical axis 2300a, 2300b, 2300c represents depth within the tissue, the horizontal axis 2310a, 2310b, 2310c denotes time and the magnitude of the measured phase-shift is represented using a color lookup table 2320 (red = positive phase-shift; blue = negative). A red horizontal line 2330a, 2330b, 2330c, at the top of each phase-shift image denotes the interval over which the laser was on. Upon initial laser exposure, a superficial region of positive phase-shift overlying a lower region of negative shift has been observed. As the laser irradiation continued, the depth at which the phase transitioned from positive to negative became progressively deeper and the magnitude of the overlying phase-shift decreased. No measurable phase-shift was detected after the laser was switched off. A protein denaturation gives rise to local microscopic structural changes and a nidus of local deformation that is detected as a phase-shift in the interferometric signal. As the laser exposure continues, the zone of active denaturation propagates in depth with overlying tissues becoming completely denatured. The depth at which the direction of the phase-shift reverses identifies the focal center of active denaturation.

To verify these results, histological sections were obtained following laser exposure and nitro-blue tetrazolium chloride (NBTC) staining was used to assess the extent of laser damage. NBTC stains positive for lactate dehydrogenase (LDH), which is a thermolabile enzyme; loss of LDH activity ensues rapidly upon heat induced cell damage and is correlated with cell lethality (as described in publications 48 and 49 identified below). Therefore, the depth of the border between unstained and stained tissue have been selected as the depth of laser damage. Corresponding phase-shift data and histology are shown in 2340a, 2340b, 2340c. The preliminary findings suggest that the border between thermally denatured tissue and viable tissue corresponds with the inflection point of the phase-shift measured with OFDI. Quantitatively, the depth-derivative of the phase-shift has been determined for each A-line and defined the depth of injury as the point of maximum negative value of the derivative. The depths determined in this way are provided in Fig. 5 as vertical lines adjacent to each M-mode image and show a good correspondence with histomorphometry.

Translating spot - Laser treatment of large epithelial surface areas can be facilitated by adding a therapeutic laser beam to the existing OFDI catheter so that the laser and OFDI beams are simultaneously scanned. The preliminary imaging studies demonstrated comprehensive esophageal imaging with an OFDI beam size of 30 $\mu$m. Obtaining a precise alignment of >1mm diameter laser beam on successive rotational scans should therefore be obtainable. To simulate the monitoring while scanning, the computer-controlled translation stage 2290 (see Fig. 4) can be controlled to repetitively toggle the sample velocity from 1.8 mm/s to 0.9 mm/s.

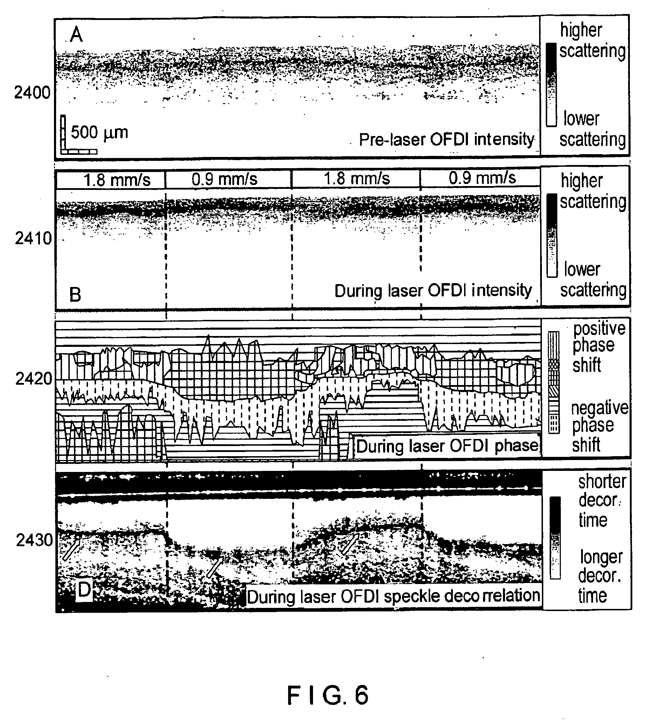

An OFDI intensity image 2400 acquired with no laser irradiation is shown in Fig. 6A. For the images 2410, 2420 and 2430 shown in Figs. 6B, 6C, and 6D, respectively, the 1450 nm laser power was about 400 mW. The translation of the samples during the exposure resulted in a line of laser damage across the surface of the sample. Since the thermal energy deposition can be proportional to the exposure time (see Eq. 1), the depth of laser damage can vary along the line according to the inverse of the translation velocity. Histology sections, obtained from regions of fast and slow translation and with an orientation perpendicular to the line, indicated laser injury depths of 0.41 mm and 0.69 mm respectively. The phase-shift data corresponding to the image 2410 of Fig. 6B is illustrated as the image 2420 in Fig. 6C. In a substantial agreement with the histomorphometric measurements, the depth of the damage determined by the phase-shift data (max negative derivative) can be 0.40 mm and 0.67 mm in the regions of fast and slow velocity, respectively.

### *Speckle Decorrelation*

Speckle is a phenomenon that is commonly observed when imaging with coherent illumination and manifests as a grainy pattern of high- and low-intensity that does not appear to correlate with the macroscopic structure. In tissue, speckle generally arises from the interference between photons that have traversed different paths during propagation within the sample. If the scatterers within the tissue are moving, even on a microscopic scale, the speckle pattern is likely seen to rapidly fluctuate. The measurements of the time-evolution of the speckle pattern can therefore provide insight into microscopic motion within the sample. This exemplary technique has been provided for investigating biomechanical properties (as described in publication 50 identified below), and thermal excitation (as described in publication 51 identified below), in biological tissues. The extension of these concepts to the depth-resolved monitoring of laser tissue interactions with OFDI has been reviewed.

Viewing the OFDI images of the tissue during laser exposure provides an indication of the potential of this exemplary technique. With no laser exposure, the speckle pattern observed in OFDI remained constant over the depth and transverse extent of the image. Under laser irradiation, the speckle pattern was observed to rapidly fluctuate in the local region of the laser beam. In slow-motion viewing, we observed that the speckle fluctuations began near the tissue surface and propagated downward in time. To quantify these observations, the rate of speckle decorrelation for each depth point of the image 2410 shown in Fig. 6B has been determined. In particular, the depth-dependent width of the temporal autocorrelation function of the OFDI intensity signal has been determined. Speckle decorrelation images were then generated by displaying the autocorrelation width using a grayscale lookup table. The image 2430 of Fig. 6D is the speckle decorrelation image corresponding to the images 2410 and 2420 of Figs. 6B and 6C, respectively. The depth of the peak decorrelation 2431 rate (black band, denoted by arrows in Fig. 6D) can be observed to vary in correspondence with the translational rate of the sample and the depth of laser damage indicated in histology. The consistency of this finding across samples of esophagus and duodenum confirm that the depth of peak decorrelation rate is a quantifiable metric for determining the depth of laser injury.

### Birefringence

As light propagates within materials, its polarization state can become altered if the index of refraction is non-isotropic. This effect is known as birefringence. Many tissues, especially muscle and collagen, exhibit strong birefringence which is lost upon thermal heating and denaturation (as described in publication 46). Polarization-sensitive OCT (PS-OCT) techniques, methods and systems have been described for quantifying burn depth through measurements of birefringence loss. (See publications 52 and 53 identified below). In PS-OCT, two detector channels can be configured to receive orthogonal polarization states of the light returning from the sample Birefringent samples induce a depth-dependent rotation of the polarization state, resulting in a variation in the percentage of the sample light detected in each channel. If the ratio of the two channels is displayed as a grayscale in a cross-sectional image, birefringence is observed as a characteristic banding pattern.

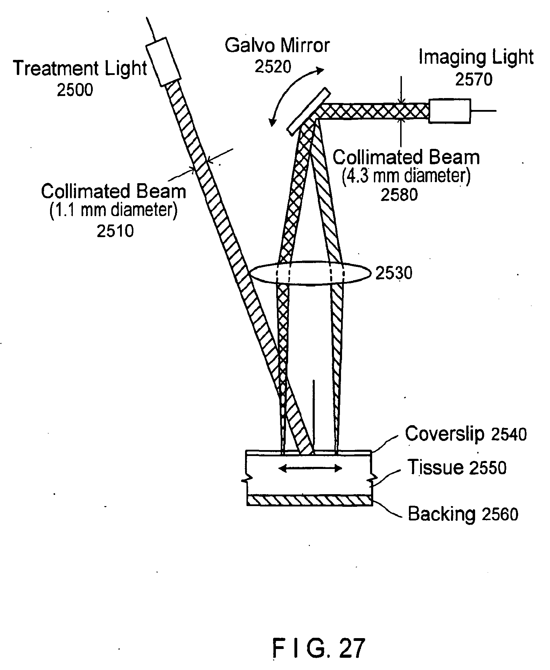

For example, the apparatus of Fig. 4 can be modified to include a galvanometric scanner so that the OFDI beam may be repetitively scanned across the surface of the tissue while the sample was held stationary and the 1450 nm laser spot remained fixed on center, as shown in Fig. 27. As shown in Fig. 27, the treatment light can be delivered through a first collimator 2500 providing a treatment beam 2510 incident on the tissue 2550 that is covered by a cover slip 2540 and against a backing 2560. The imaging light may be provided by a second collimator 2570 producing an imaging beam 2580 that is directed by a galvo-mirror 2520 through a lens 2530. This arrangement/system can be an exemplary embodiment of a therapeutic monitoring system applicable to applications in dermatology. OFDI images or video of esophageal and duodenal tissues were acquired during laser irradiation.

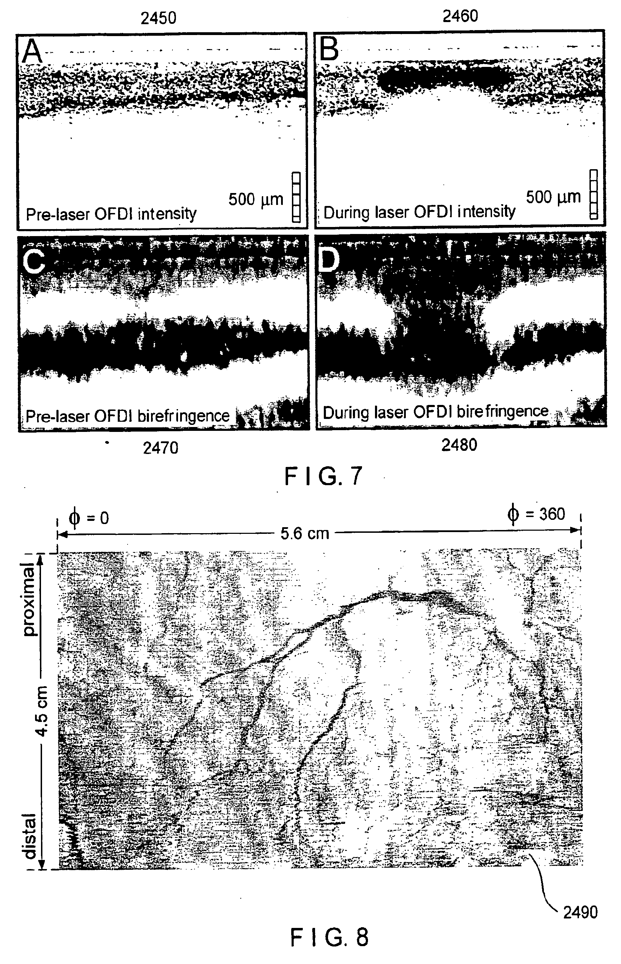

Figs. 7A-7D show images of the representative data. In frames acquired prior to laser irradiation, the layered esophageal structure can be observed in the intensity image 2450 (see Fig. 7A) and characteristic birefringence banding can be observed in the corresponding polarization image 2460 (see Fig. 7B). In frames acquired during laser exposure, the epithelial scattering intensity may be increased dramatically within the 1.1 mm laser spot 2470 (see Fig. 7C), and the birefringence banding in the corresponding polarization image 2480 (see Fig. 7D) can be lost. Reviewing the polarization moving images in slow-motion, a zone of decreased birefringence can be observed that may begin near the surface and propagated downward. These observations are generally consistent with a downward propagating zone of denatured tissue. Measurements of the percent-loss of birefringence is a quantitative metric for monitoring laser thermal damage.

### Scattering

Thermally induced changes to the microscopic structure of tissue can alter optical scattering. Since the signal in OFDI arises from scattering and small changes can be detected over a large dynamic range, we investigated the use of scattering measurements for monitoring thermally induced changes in tissue. Scattering changes observed in image 2460 of Fig. 7B may be representative of the preliminary observations in both duodenum and esophagus samples. In certain cases, it was determined that the significant scattering changes within the epithelium and relatively smaller changes in the underlying tissues of the muscularis mucosa and muscularis propria. For example, two potential quantitative metrics for laser damage that could be obtained from scattering measurements: changes in the depth-resolved scattering intensity and changes in the depth-integrated scatting intensity.

### Blood flow

Laser therapy can to alter vessels and capillaries resulting in decreased blood flow (as described in publication 54 identified below). Since the esophageal mucosa is highly vascularized, monitoring changes in blood flow may provide an additional method for monitoring laser therapy. An image 2490 of Fig. 8, acquired during our recent swine studies, graphically illustrates the porcine esophageal vascularity. This exemplary image 2490 was generated by unwrapping the tubular image data to display the epithelial surface as if the esophagus was longitudinally opened and pinned flat. The intensity data has been integrated over depth into the tissue. Although this type of large scale visualization is a convenient way to map the vessels, it is possible to use a more sensitive and quantitative method/technique/system for measuring blood flow. Doppler OCT (as described in publications 55 and 56 identified below) has been demonstrated for visualizing and quantifying blood flow in tissue and has been investigated as an arrangement for assessing flow following laser therapy (as described in publication 57 identified below). The Doppler measurements with OFDI (as described in publication 24 identified below) have been described, and the possibility of simultaneously measuring structure and flow in vivo has been reviewed.

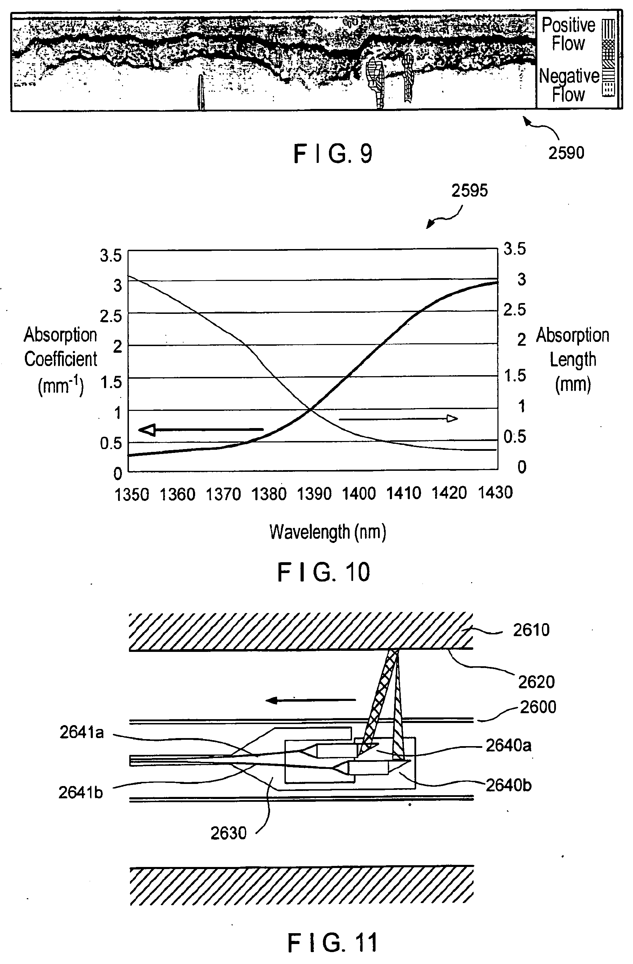

A cross-sectional view of an exemplary image 2590 of Fig. 9 has been acquired in the esophagus of a living swine and displays intensity as a grayscale and Doppler as a superimposed color. The coordinates $(r,\theta)$ of this data have been mapped to Cartesian coordinates (vertical, horizontal) for simplicity of display. This result was representative of our observations at multiple locations in two swine. Additionally, in time-sequences of Doppler images, we clearly observed pulsatile flow.

| Summary of Monitoring | | |
|---|---|---|
| Cause | Effect | Measurement |
| Thermal denaturing of cellular proteins and collagen | Focal deformation | Phase & Speckle |
| | Loss of birefringence | Polarization |
| | $\Delta$ Scattering | Intensity |
| Thermal coagulation of vessels | Loss of blood flow | Doppler flow & Vascular Map |

Based on the preliminary investigation, the proposed measurements would likely be complementary: and the phase-shift and speckle decorrelation, which are only applicable during laser irradiation, may be more sensitive and provide greater spatial resolution. The changes in birefringence, scattering and flow are persistent and could be applied for follow-up imaging after laser treatment.

*Exemplary Control*

In addition to monitoring for laser thermal injury, effective conformal laser therapy may use precise control over the volume of treated tissue. One exemplary approach to controlling treatment depth is to operate within the conditions for thermal confinement in order to minimize collateral damage and to manipulate laser wavelength, power, and exposure time to control the depth of thermal injury. In the transverse dimension (along the epithelial surface), thermal injury can be controlled through the use of a raster-scanned, spatially-collimated beam. A flat-top beam with a diameter of 1-3 mm with well-defined edges may allow spatial control while also permitting therapy of large epithelial areas through raster scanning. Exemplary laser control parameters are described herein below in the context of Eq. 1. The temperature distribution of Eq. 1 generally applies only in the limit of weak scattering.

*Wavelength*

From the temperature distribution of Eq. 1, it is evident that $\mu_a$ would likely be an optimal parameter for control over depth of laser injury. Although $\mu_a$ is characteristic of the sample rather than an externally controllable parameter, in this invention we exploit the wavelength dependence of $\mu_a$ to achieve depth control. In this invention, we target the absorption coefficient at longer wavelengths where water absorption dominates. Since the water content is approximately constant in epithelial tissues, thermal injury depth can be closely regulated by changing the laser wavelength by small amounts. In the vicinity of the water absorption band near 1.45 $\mu$m, absorption lengths (see graph 2595 of Fig. 10) range from 0.3 mm to over 2 mm within a narrow spectral range (1375 nm to 1430 nm). These lengths correspond well to the characteristic length scales appropriate for the treatment of epithelial disease. A tunable laser, operable in the vicinity of the 1450 nm water absorption band can be used to control therapy through wavelength tuning.

*Power and Exposure Duration*

Upon the review of Eq. 1, the absorption coefficient does more than control the exponential depth decay of the temperature distribution; e.g., it also can control the amplitude. Since the amplitude term is also dependent upon power density and exposure duration, these variables can be used to normalize the amplitude while allowing the absorption coefficient to change.

*Procedure Duration*

In the evaluation of a proposed new therapy, it may be important to estimate the preferable procedure time and evaluate this estimate in the context of competing approaches and constraints specific to the clinical setting and patient acceptance. PDT is currently applied for the treatment of BE in the endoscopy setting and requires procedure times on the order of 20 minutes. For the exemplary conformal laser therapy technique, the procedure performance time may be estimated by $2At/(\pi rv)$, where At is the treatment area, r is the laser spot radius, and v is the laser spot scan velocity. For an esophageal treatment length of 60 mm and an esophageal diameter of 20 mm.
According to the exemplary embodiment of the present invention, a combined system can be provided which may allow for a controlled laser excitation. In one exemplary embodiment, the exemplary system can be used endoscopically for conformal laser therapy capable of comprehensively treating epithelial lesions while minimizing collateral damage to adjacent tissues.

*Exemplary System Design*

According to the exemplary embodiment of the present invention, a system can be provided for performing conformal laser therapy of epithelial disease through a combination of monitoring and control. Since laser beams can easily be shaped and spatially scanned and since margins in the transverse plane (along the surface of the esophagus) are less critical, the primary challenge for achieving accurate control of laser therapy is in limiting and adjusting the depth of laser damage. Based on the modeling and analysis described above, it is possible to utilize laser wavelength and power and scanning speed to vary the depth of laser damage over a clinically significant range while not significantly altering the transverse extent of injury.

*Exemplary Therapy Laser Arrangement*

The laser wavelengths between approximately 1375 nm and 1430 nm can provide absorption lengths ranging from over 2 mm to less than 0.3 mm. Semiconductor lasers can operate in this spectral range. Since such lasers can be compact and environmentally stable, these laser can be effectively used in clinical applications. Materials suitable for this specific wavelength range, however, may not be standard. A less expensive alternative for the early testing phase of exemplary embodiments of the methods according to the present can be provided by a solid-state laser material, tetravalent chromium-doped YAG (Cr4+:YAG). For example, a tunability with this material over the spectral range of 1340 nm - 1570 nm can be implemented (as described in publications 58 and 59 identified below). The exemplary design and construction of tunable solid-state lasers that operate in the near infrared spectral range are described in publications 60-65 identified below. An electromechanical shutter, external to the laser resonator, can be used to turn on/off the exemplary laser.

*Exemplary Benchtop System*

An exemplary embodiment of a benchtop optical system according to the present invention may be provided that can be similar to the systems shown in Figs. 4 and 27 and described herein. For example, the OFDI sampling beam may be focused at the sample to a diameter of ~25 $\mu$m. The axial location of the focus can be determined using a standard z-scan technique, and may be registered within the OFDI cross-sectional image. The subsequent axial positioning of the samples within the OFDI image window may ensure a constant focus location for all samples. Data may be collected with the two beams fixed with respect to each other and while the sample is translated perpendicular to the laser beam axis.

*Exemplary Positioning and Registration of Laser and OFDI beams*

According to the exemplary embodiment of the present invention, the offset between the OFDI beam and the center of the laser spot is not critical for monitoring. OFDI data may be collected for various offsets (as depicted in Fig. 4) to determine the offset that yields the greatest indicated depth of thermal injury. This offset can then be used in all subsequent studies and may be registered as follows. A small, low-power, short-duration epithelial burn may be induced on the surface of the sample while the sample is held fixed (non-translating). As shown in Fig. 7, the increase in epithelial scattering can be readily observed in OFDI and is spatially localized as defined by the laser beam profile. Although not illustrated in Fig. 4, the OFDI beam can be relayed to the focusing lens by a pair of galvanometers that provide two-dimensional scanning. The galvanometers may be used to generate an en face OFDI image of the sample and the epithelial burn may appear as a circle of increased scattering. The galvanometers can then be positioned and fixed so that the OFDI beam is positioned with the desired offset (as schematically shown in Fig. 4).

*Exemplary Wavelength Scaling*

One of the purposes of this experiment is to test the exemplary technique and method of wavelength variation and power normalization according to the present invention for achieving clinically relevant variation in the depth of laser damage. Laser wavelength may be varied from about 1375 nm to 1405 nm in 2 nm steps with laser spot size and scanning speed held constant. For each wavelength, the laser power may be adjusted so that the product $P_d \oplus \mu_a$ in Eq. 1 can be maintained as constant. This should yield lines of constant width and with damage depth ranging from approximately 0.25 to 1.5 mm.

*Exemplary Scanning Velocity Scaling*

One exemplary embodiment according to the present invention for affecting therapeutic depth may include scaling the scan velocity. For example, the therapy beam scan speed can be varied from 1 mm/s to 5 mm/s. Slower scan speeds allow time for heat to conduct to deeper areas of the tissue, producing deeper therapy.

*Exemplary Positioning and Registration of Laser and OFDI Beams*

To ensure accurate therapeutic monitoring, the spatial relationship between the OFDI sampling beam and the laser spot can be controlled.

*Exemplary Endoscopic Probe Designs*

One exemplary embodiment of the present invention can include an endoscopic probe for comprehensive, volumetric imaging and simultaneous laser therapy, as shown in Fig. 11. For example, two beam relay optics 2640a and 2640b may be used, one of which conveys imaging light 2640b and the other therapy light 2640a. These relay optics are placed within a housing 2630 that is enclosed within a first transparent sheath 2600. A balloon centration mechanism (as described above) 2620 may be used to maintain a constant distance between the optical probe 2630 and the tissue surface 2610. Laser light and the OFDI beam may be delivered through separate optical fibers 2641a and 2641b. Each fiber may have its own relay optics to produce independently controllable spot sizes. A further exemplary embodiment of the present invention can include these relay optics designed to produce overlapping spots. The optical fibers and distal optics may be housed in a wound-wire drive shaft and placed inside a balloon-centering probe identical to the balloon sheaths.

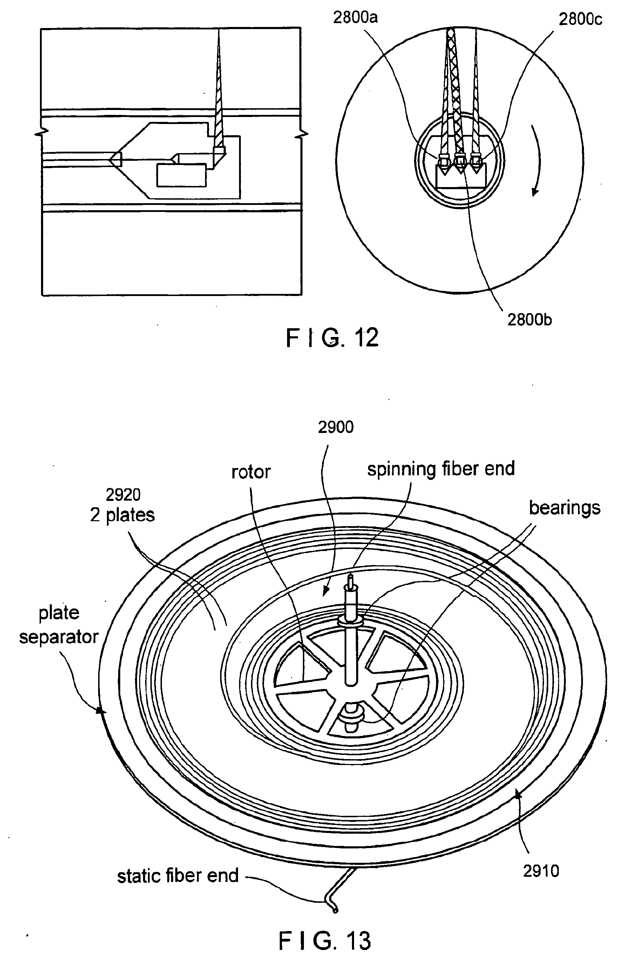

Longitudinal scans can be activated using a computer controlled translation stage attached to the proximal end of the drive shaft. This exemplary arrangement may be the same as the arrangement which can be used for the pull-back esophageal imaging of our preliminary studies. A manual rotation of the drive shaft may be possible, as is automated rotation using an exemplary rotary coupler 2900 shown in Fig. 13. In one exemplary embodiment of the present invention, an endoscopic system may screen for disease over large fields-of-view, accurate monitoring of laser-tissue interaction, and precisely control laser therapy. One of the applications of such exemplary embodiment may be the identification and treatment of epithelial cancers and their precursors. In a further exemplary embodiment, the system can incorporate procedures and software modules than can directly link screening, monitoring, and control.

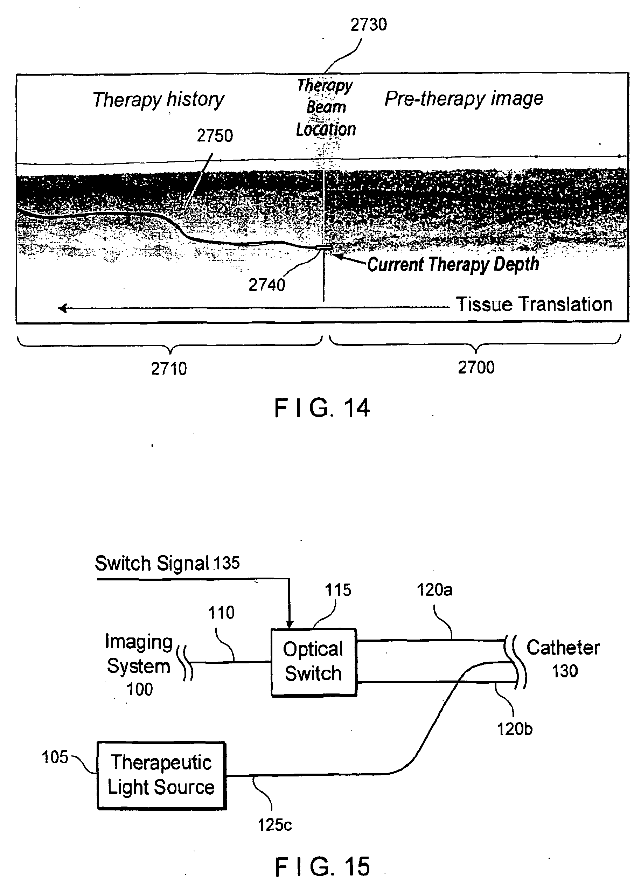

In yet another exemplary embodiment, the system may be used to generate a high-resolution, 3-dimensional map of the entire distal esophagus to facilitate therapeutic planning. Thereafter, the use may be presented with a 'live' cross-sectional image comprising three sections, as illustrated in Fig. 14. A right section 2700 of the image may be the tissue immediately ahead of the therapeutic lase, the center 2730 of the image may be the location of the laser with a marker 2740 designating the zone of therapy, and the left section 2710 of the image may be the tissue that has already been treated. Since the three beams may be continuously scanning, the tissue may appear to move from right to left as the image updates. The user (e.g., an endoscopist) may operate a control servo to start/stop the treatment and increase or decrease the depth of therapy. By viewing the zone of treatment 2710 and looking ahead to the untreated tissue 2700, the user may be able to steer and conform the region of laser therapy to the desired target.

An exemplary embodiment of the endoscopic probe for imaging, monitoring and laser therapy through a centering balloon according to the present invention is shown in Fig. 12. This exemplary probe can rotate to scan the esophagus circumferentially and may be longitudinally translated at a slower rate to define segments for therapy. This probe may include, e.g., three or more optical channels: a first channel 2800c for imaging the tissue prior to laser irradiation, a second channel 2800b for treatment, and a third channel 2800a for monitoring. Each optical fiber may be separately imaged transversely onto the esophageal wall through the balloon. The alignment of the resulting output beams may be such that, upon rotation in the clockwise direction, the imaging beam precedes the treatment beam sufficiently so that non-treated tissue may be sampled. The monitoring beam may be aligned to fall within the laser spot. Following initial alignment of the three beams, the optics may be bonded together with epoxy, and the alignment may be fixed.

*Exemplary Rotary Junction*

An exemplary rotational coupler according to the present invention which can connect the three-channel catheter to the OFDI system is shown in Fig. 13, and can be referred to "watch-spring" rotary junction (since it can rely on two concentric spools). For example, as the inner spool 2900 rotates in one direction, optical fiber is wound from the outer spool 2910 onto the inner spool 2900. On reversing the direction, the fiber can unwind from the inner spool. Ribbon optical fiber may be used and two parallel plates 2920 with a gap matched to the ribbon width can ensure that the windings remain flat and do not bind. The plates may be sufficiently large so that up to, e.g., 100 rotations may be possible prior to requiring counter rotation. With a 1 mm laser spot, full treatment of 6 cm long esophageal segments may be 60 revolutions. A plate diameter of less than 10 cm can be used. In addition to accommodating three optical channels, this exemplary embodiment of the arrangement and system according to the present invention can avoid the loss and back-reflections that arise from air-gap couplers.

*Exemplary High-speed Acquisition and Processing*

A further exemplary embodiment of the system and arrangement according to the present invention can utilize, e.g., a high-speed imaging system. The exemplary embodiment of the digital acquisition and processing system can be based on VME-bus hardware for acquiring, processing and storing the OFDI signals in real-time. The exemplary components of such exemplary system and arrangement may comprise a VME chassis containing high-speed digitizers residing on a single-board computer and fiber-optic links to a RAID storage array. This exemplary system and arrangement can be controlled via a host processor (e.g., a personal computer). The analog OFDI signals may be digitized using wideband receivers (e.g., 12 bit, 210 MS/s) with integrated field-programmable-gate-array (FPGA) processors. Processing power, resident on the acquisition board, may be importance since the raw data rate may be 800 MB/s for the two polarization channels of the OFDI system. The FPGA processor can be configured or programmed to transform each polarization channel from the frequency-domain to a 1024-element array representing reflectivity versus depth (one A-line). This data can be passed to the single-board computer for subsequent processing and for combining the two channels prior to transferring the final data to a RAID array of hard drives. The final data storage rate may be, e.g., 400 MB/s. By striping the data across multiple hard drives, this data rate can be continuously sustained.

Software on a processing arrangement in accordance with an exemplary embodiment of the present invention can permit a user control over the exemplary system, and may enable a display of the images at a down-sampled rate in real-time. For example, the exemplary system can be used in two exemplary modes: a burst mode at full data rate, and continuous mode at half data rate. The exemplary endoscopic system and arrangement can include the components and software described above, and additional procedures (e.g., software) can be provided to program both the FPGA processor and single-board computer to facilitate the computations of phase-shift, birefringence, speckle, and Doppler signals in real-time. The combined computational capacity of the Vertex 4 Pro FPGA and quad G4 single-board computers may be ample for displaying the monitoring signal in real-time.

*Exemplary Laser*

Using Eq. 1, the spot size while maintaining a constant scan velocity can be doubled by using a 4-fold increase in the laser power in order to maintain a constant temperature distribution in the tissue. Doubling the scan velocity at a constant spot size should use a doubled laser power. One exemplary embodiment of a laser arrangement in accordance with the present invention can utilize a single-emitter semiconductor laser diode. Previous devices have provided more than 3 W of laser power over this spectral range using a simple external cavity design including a diffraction grating for wavelength control. The laser power and wavelength may be controlled via the host processing arrangement of the OFDI system based on an analog signal from a potentiometer. The potentiometer may be a hand-held dial that the user (e.g., an endoscopist) may use to increase or decrease the depth of laser damage.

*Exemplary User Interface*

The exemplary embodiment of the system and method according to the present invention can provide a user interface to the operator that includes a cross-sectional image of the tissue. The image may be continuously updated and may include views of treated and upcoming, untreated tissue as well as a designation for the zone of laser treatment as determined by the monitoring procedures. The user interface may be programmed on the host processing arrangement, and can use computational results from the FPGA processor and single-board computer. Images and laser parameters may be archived to the RAID array.

In one further exemplary embodiment of the present invention, the imaging system/arrangement 100 can be connected to a three-fiber probe using an optical switch 115 as shown in a block diagram of Fig. 15. The exemplary probe, such as that described above with reference to Fig. 12, can include two imaging fibers and one therapy fiber. The switch 115 can alternately couple imaging light to one of the two imaging fibers 120a, 120b which can be used to acquire re-therapy images and, e.g., during-therapy imaging. A therapeutic light source 105 may connect directly to the therapy fiber 125c. The fibers can be connected to the catheter 130, which can be, e.g., the exemplary catheter shown Fig. 12. A signal from the imaging system 100 can control the state of the optical switch 115.

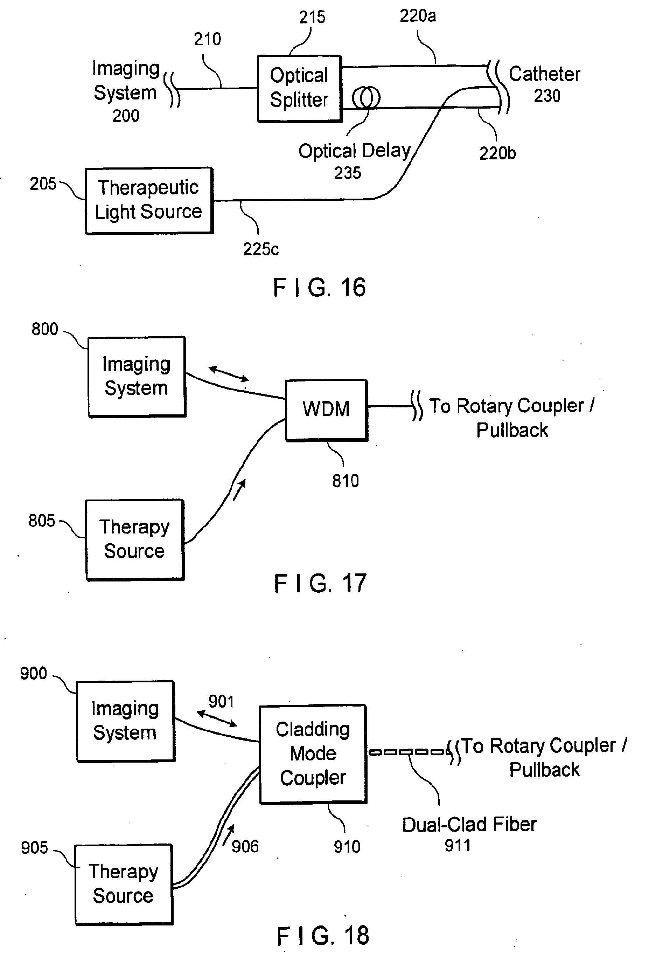

In yet another exemplary embodiment according to the present invention shown in Fig. 16, the exemplary imaging system/arrangement 200 can be coupled to an exemplary three-port catheter such as one shown in Fig. 12 via an optical splitter 215 that can couple light to both of two imaging fibers 220a, 220b. This exemplary imaging system can separate the image signal from each using path-length encoding techniques. To generate the differential path length, an optical delay 235 may be placed in one fiber 220b or multiple fibers. The therapeutic light source 205 can be coupled directly or indirectly to the therapy fier 225c of the catheter.

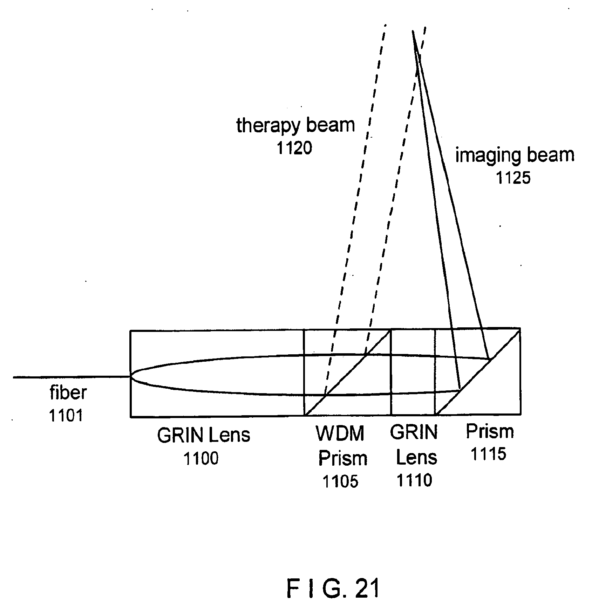

In still another exemplary embodiment of the exemplary imaging system/arrangement 800 according to the present invention shown in Fig. 17, light may be combined with the therapy source 805 using a single wavelength-division multiplexer 810. The combined light may be coupled to a single fiber rotary coupler, and then to an exemplary single fiber catheter such as the catheter shown in Fig. 21.

In a further exemplary embodiment of the imaging system/arrangement 900 according to the present invention shown in Fig. 18, light may be combined with the therapy light 905 using a cladding mode coupler that couples the imaging system 900 light from the single mode fiber 901 to the single mode core of a dual-clad fiber 911 and the therapy light from a multimode fiber 906 to the cladding mode of a dual-clad fiber 911.

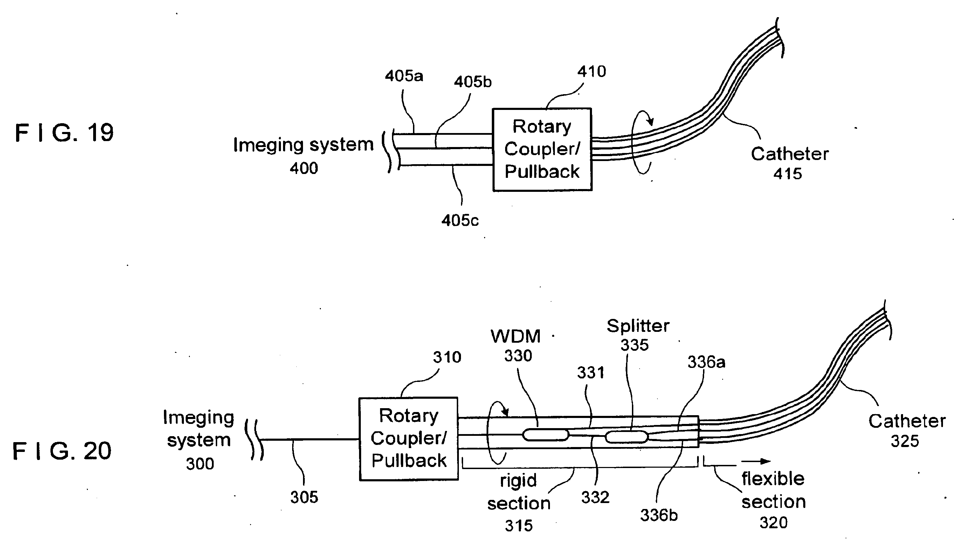

Fig. 19 shows exemplary connections between a system 400 with three output fibers 405a, 405b, 405c, such as one shown schematically in, e.g., Figs. 15 and 16, and a three-port catheter 415, such as one shown in Fig. 12 via a multi-channel rotary coupler 410, such as one shown in Fig. 13.

Fig. 20 shows a schematic diagram of an exemplary system 300 according to the present invention in which a single fiber 305 containing both the imaging light and therapy light may be coupled to a single-channel rotary coupler 310. For example, after the rotary coupler 310, the light can be divided by a wavelength-division multiplexer (WDM) 330 that separates the imaging light onto a first fiber 332 and the therapy light onto a second fiber 331. The imaging light may further be separated using an optical splitter 335 that greats two imaging ports 336a and 336b. The fibers 31, 336a, 336b can be connected to a three-port catheter 325design such as that shown in Fig. 12. The catheter section 320 may be flexible allowing endoscopic insertion and the section containing a WDM 330 and a splitter 335 can be enclosed within a rigid tube 315 to protect these components.

Fig. 21 shows a side view of an exemplary embodiment of a distal optics arrangement according to the present invention that may create a single imaging beam 1125 and a separate therapy beam 1120 from a single-mode fiber 1101. For example, the light from the fiber containing both imaging and therapy light can first be focused by a first GRIN lens 1100. The light is then passed into a wavelength-division multiplexing prism 1105 that can direct the therapy light wavelengths upward to create the therapy beam 1120, and transmits the imaging light wavelengths to a second GRIN lens 1110, which can alternately focus the imaging light and directs it to a final prism 1115 that directs the imaging beam 1125 upward. The angle of the prisms 1105 and 1115 may be such that the beams are made to overlap at the appropriate distance from the device.

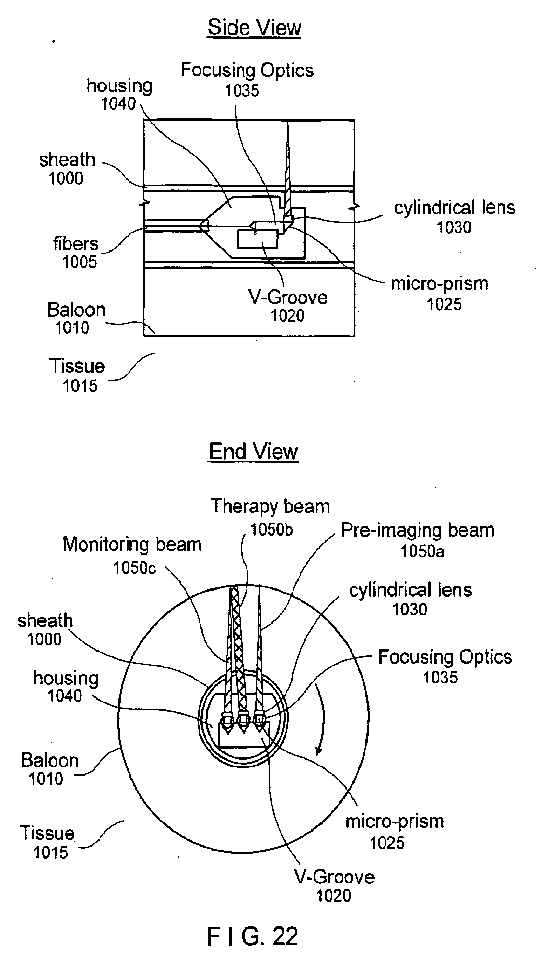

Fig. 22 shows side and front views of an exemplary embodiment of a three-port catheter in accordance with the present invention. The exemplary catheter can include three fibers 1005 that connect to three sets of focusing optics 1035 contained in a V-groove 1020 inside a housing 1040. The focusing optics can provide beam focusing. Micro-prisms 1025 can redirect the optical beam upward through a cylindrical lens 1030 that corrects for astigmatism induced by the transparent sheath 1000. A balloon 1010 centration mechanism may be used to maintain centering of the optics 1035 within the luminal tissue 1015. In the end-view, the monitoring beam 1050c, therapy beam 1050b, and pre-imaging beam 1050a can be seen. The housing 1040 can be adapted to rotate by a multichannel rotary coupler such as one shown in Fig. 13.

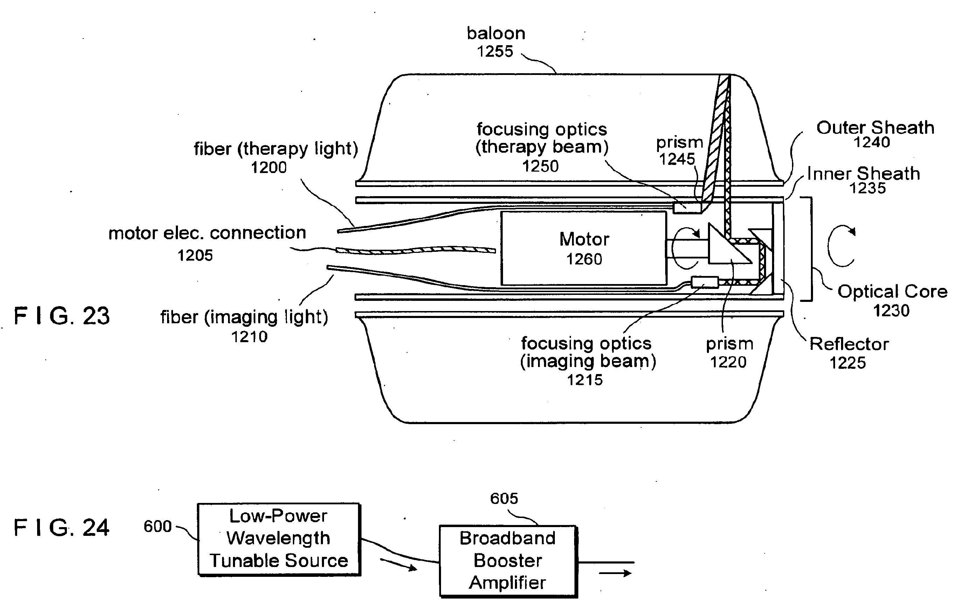

Fig 23 shows a side view of an exemplary embodiment of a catheter in accordance with the present invention which can utilize a miniature motor 1260 to achieve rotation of the imaging beam. For example, the motor 1260 can be enclosed within a transparent sheath 1235. The rotation of the motor shaft can rotate a prism 1220. The imaging light can be coupled to the distal optics via a fiber 1210, where the light can be focused by focusing optics 1215, and reflected onto the prism 1220 by a reflector 1225. The rotation of the prism 1220 sweeps the imaging beam circumferentially. The motor electrical connections 1205 can be achieved through the same lumen as the fiber 1210. The therapy light is coupled to the distal optics on fiber 1200. This therapy light may be focused using focusing optics 1250, and directed sideways by prism 1245 at a fixed rotational angle relative to the inner sheath 1235. The imaging beam thus sweeps through the fixed therapy spot. The translation of the therapy spot is achieved by rotation of the entire inner sheath 1235 within the outer sheath 1240. This exemplary rotation can be achieved through the use of a multi-channel rotary coupler such as a coupler shown in Fig. 13. The catheter can use a balloon 1255 for centration of an optical core 1230.

Fig. 24 shows a block diagram of an exemplary embodiment of a laser therapy source according to the present invention with wavelength tunability utilizing a low power wavelength tunable source 600, followed by a broadband booster amplifier 605 to increase the optical power.

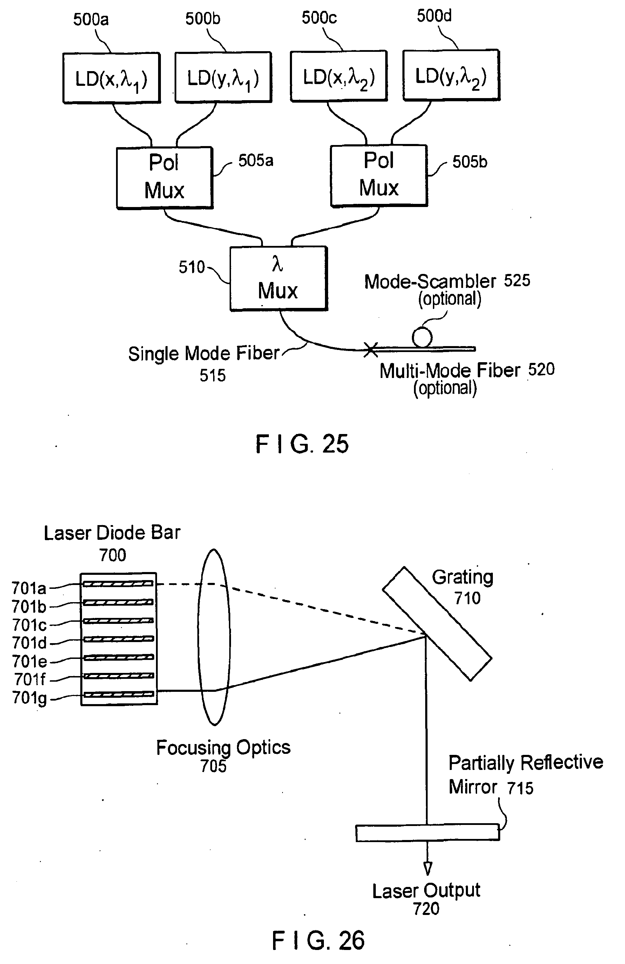

Fig. 25 shows a block and functional diagram of an exemplary embodiment of a laser therapy source incorporating multiple laser diodes 500a, 500b, 500c, 500d at difference wavelengths and polarizations, and the exemplary procedure to implement such arrangement. For example, the light can be combined by the polarization multiplexers 505a, 505b and wavelength division multiplexers 510 to a single mode fiber 515. Optionally, the light can be coupled to a multimode fiber 520. A fast mode scrambler 525 can be used to scramble the transverse mode pattern output from the multi-mode fiber at a fast rate. Other source arrangements which can output light on a single mode fiber can use a similar design to couple light to a multimode fiber.

Fig. 26 shows an exemplary embodiment of a therapy light source and use thereof according to the present invention. For example, a laser diode bar 700 can be used with multiple wavelengths 701a-g. Each waveguide can be coupled to a free-space laser cavity through a lens apparatus 705 and a grating 710 and a partially reflecting end mirror 715. Because of the wavelength dispersion of the grating, the laser formed by each waveguide lases at a different wavelength. Thus, by adjusting the drive current to each of the waveguides 701a-g, the laser output 720 power and spectral shape can be adjusted.

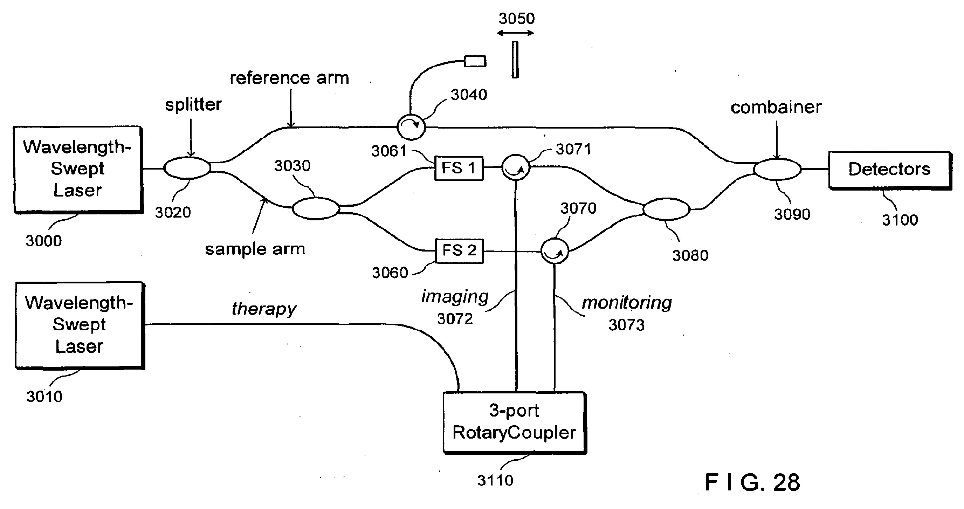

In a further exemplary embodiment according to the present invention, a single OFDI system can be modified to facilitate a detection of both the imaging and monitoring signals through the use of acousto-optic frequency shifters as shown in Fig. 28. For example, a wavelength swept laser source 3000 can be separated by a first splitter 3020 to produce a sample arm path and reference arm path. The sample arm path is further separated by a second splitter 3030, with a first output of this splitter being directed to a first frequency shifter 3061 and a second output being directed to a second frequency shifter 3060. Each of the frequency shifters can be driven with a separate frequency. The light from the first frequency shifter 3061 can be coupled through an optical circulator 3071 to the imaging fiber 3072 of a three-fiber rotary coupler 3110 like that shown in Fig. 13. The light from the second frequency shifter 3060 may be coupled through a circulator 3070 to a monitoring fiber 3073 of the same rotary coupler.

A separate therapy laser 3010 can be coupled to the third therapy fiber. The returned light on the imaging fiber 3072 and monitoring fiber 3073 may be recombined at an optical combiner 2080, and mixed with the reference arm light at a second combiner 3090 with the output directed to a set of detectors 3100. Due to the frequency shifters, the interference signal due to the imaging light and the interference signal due to the monitoring light are encoded at separate carrier frequencies and can be separated through conventional frequency domain techniques.

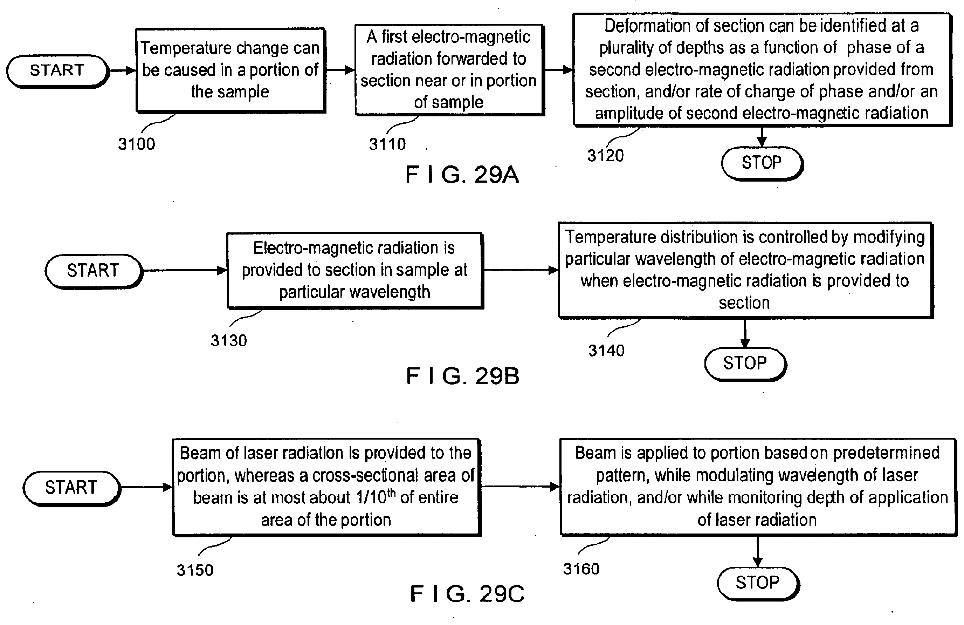

Fig. 29A shows a flow diagram of an exemplary embodiment of a method for obtaining information associated with at least one portion of a sample according to the present invention. For example, a temperature change can be caused in the portion of the sample in step 3100. At least one first electro-magnetic radiation can be forwarded to a section near or in the portion of the sample in step 3110. A deformation of the section can be identified at a plurality of depths as a function of (i) a phase of at least one second electro-magnetic radiation provided from the section, and/or (ii) a rate of change of the phase and/or an amplitude of the second electro-magnetic radiation in step 3120.

Fig. 29B shows a flow diagram of another exemplary embodiment of the method for controlling a temperature distribution in the sample according to the present invention. For example, an electro-magnetic radiation can be provided to the section in the sample at a particular wavelength in step 3130. The temperature distribution can be controlled by modifying the particular wavelength of the electro-magnetic radiation when the electro-magnetic radiation can be provided to the section in step 3140.

Fig. 29C illustrates a flow diagram of yet another exemplary embodiment of the method for applying a laser radiation to at least one portion of a biological structure according to the present invention. For example, a beam of the laser radiation can be provided to the portion in step 3150, whereas a cross-sectional area of the beam is at most about 1/10th of an entire area of the at least one portion. In step 3160, the beam can be applied to the portion (I) based on a predetermined pattern, (II) while modulating a wavelength of the laser radiation, and/or (III) while monitoring a depth of the application of the laser radiation.

*Exemplary References*

1. Devesa SS, Blot WJ and Fraumeni JF, Jr. Changing patterns in the incidence of esophageal and gastric carcinoma in the United States. Cancer 1998;83:2049-2053.

2. Barr H, Stone N and Rembacken B. Endoscopic therapy for Barrett's oesophagus. Gut 2005;54:875-884.

3. Johnston MH. Technology insight: ablative techniques for Barrett's esophagus - current and emerging trends. Nature Clinical Practice Gastroenterology & Hepatology 2005;2:323-330.

4. Falk GW, Chittajallu R, Goldblum JR, Biscotti CV, Geisinger KR, Petras RE, Birgisson S, Rice TW and Richter JE. Surveillance of patients with Barrett's esophagus for dysplasia and cancer with balloon cytology. Gastroenterology 1997;112:1787-1797.

5. Spechler SJ. Barrett's esophagus: should we brush off this ballooning problem? Gastroenterology 1997;112:2138-2142.

6. Kubba AK, Poole NA and Watson A. Role of p53 assessment in management of Barrett's esophagus. Dig Dis Sci 1999;44:659-667.

7. Reid BJ. p53 and neoplastic progression in Barrett's esophagus. Am J Gastroenterol 2001;96:1321-1323.

8. Sharma P, Weston AP, Topalovski M, Cherian R, Bhattacharyya A, Sampliner RE. Magnification chromoendoscopy for the detection of intestinal metaplasia and dysplasia in Barrett's oesophagus, GUT 2003;52: 24-27.

9. Kuipers EJ and Haringsma J. Diagnostic and therapeutic endoscopy. Journal of Surgical Oncology 2005;92:203-209.

10. Georgakoudi I, Jacobson BC, Van Dam J, Backman V, Wallace MB, Muller MG, Zhang Q, Badizadegan K, Sun D, Thomas GA, Perelman LT and Feld MS. Fluorescence, reflectance, and light-scattering spectroscopy for evaluating dysplasia in patients with Barrett's esophagus. Gastroenterology 2001;120:1620-1629.

11. Adrain AL, Ter HC, Cassidy MJ, Schiano TD, Liu JB and Miller LS. High-resolution endoluminal sonography is a sensitive modality for the identification of Barrett's metaplasia. Gastrointest Endosc 1997;46:147-151.

12. Canto MI. Vital staining and Barrett's esophagus. Gastrointest Endosc 1999;49:S12-16.

13. Huang D, Swanson EA, Lin CP, Schuman JS, Stinson WG, Chang W, Hee MR, Flotte T, Gregory K, Puliafito CA and Fujimoto JG. Optical coherence tomography. Science 1991;254:1178-1181.

14. Tearney GJ, Brezinski ME, Bouma BE, Boppart SA, Pitvis C, Southern JF and Fujimoto JG. In vivo endoscopic optical biopsy with optical coherence tomography. Science 1997;276:2037-2039.

15. Evans JA, Poneros JM, Bouma BE, Bressner J, Halpern EF, Shishkov M, Lauwers GY, Mino-Kenudson M, Nishioka NS and Tearney GJ. Optical Coherence Tomography to Identify Intramucosal Carcinoma and High Grade Dysplasia in Barrett's Esophagus. Clinical Gastroenterology and Hepatology 2005;4:38-43.

16. Poneros JM, Brand S, Bouma BE, Tearney GJ, Compton CC and Nishioka NS. Diagnosis of Specialized Intestinal Metaplasia by Optical Coherence Tomography. Gastroenterology 2001;120:7-12.

17. Brand S, Poneros JM, Bouma BE, Tearney GJ, Compton CC and Nishioka NS. Optical Coherent Tomography in the Gastrointestinal Tract. Endoscopy 2000;32:796-803.

18. de Boer JF, Cense B, Park BH, Pierce MC, Tearney GJ and Bouma BE. Improved signal-to-noise ratio in spectral-domain compared with time-domain optical coherence tomography. Optics Letters 2003;28:2067-2069.

19. Choma MA, Sarunic MV, Changhuei Y and Izatt JA. Sensitivity advantage of swept source and Fourier domain optical coherence tomography. Optics Express 2003;11:2183-2189.

20. Leitgeb R, Hitzenberger CK and Fercher AF. Performance of Fourier domain vs. time domain optical coherence tomography. Optics Express 2003;11:889-894.

21. Yun SH, Tearney GJ, de Boer JF, Iftimia N and Bouma BE. High-speed optical frequency-domain imaging. Optics Express 2003; 11:2953-2963.

22. Yun SH, Boudoux C, Tearney GJ and Bouma BE. High-speed wavelength-swept semiconductor laser with a polygon-scanner-based wavelength filter. Optics Letters 2003;28:1981-1983.

23. Oh WY, Yun SH, Tearney GJ and Bouma BE. 115 kHz tuning repetition rate ultrahigh-speed wavelength-swept semiconductor laser. Optics Letters 2005;30:3159-3161.

24. Vakoc BJ, Yun SH, de Boer JF, Tearney GJ and Bouma BE. Phase-resolved optical frequency domain imaging. Optics Express 2005;13:5483-5493.

25. Brown SB, Brown EA and Walker I. The present and future role of photodynamic therapy in cancer treatment. Lancet Oncol 2004;5:497-508.

26. van den Boogert J, van Hillegersberg R, Siersema PD, de Bruin RW and Tilanus HW. Endoscopic ablation therapy for Barrett's esophagus with high-grade dysplasia: a review. Am J Gastroenterol 1999;94:1153-1160.

27. Sampliner RE, Fennerty B and Garewal HS. Reversal of Barrett's esophagus with acid suppression and multipolar electrocoagulation: preliminary results. Gastrointest Endosc 1996;44:532-535.

28. Sampliner RE. Endoscopic ablative therapy for Barrett's esophagus: current status. Gastrointest Endosc 2004;59:66-69.

29. Soetikno RM, Gotoda T, Nakanishi Y and Soehendra N. Endoscopic mucosal resection. Gastrointest Endosc 2003;57:567-579.

30. Ganz RA, Utley DS, Stern RA, Jackson J, Batts KP and Termin P. Complete ablation of esophageal epithelium with a balloon-based bipolar electrode: a phased evaluation in the porcine and in the human esophagus. Gastrointest Endosc 2004;60:1002-1010.

31. Mark H. Johnston, Brooks D. Cash, Cathy A. Dykes, Halisha S. Mays and Lavonne R. Johnston Cryoablation of Dysplasia in Barrett's Esophagus (BE) and Early Stage Esophageal Cancer. Gastrointest Endosc 2006 ;63: AB223.

32. Overholt BF, Panjehpour M and Haydek JM. Photodynamic therapy for Barrett's esophagus: follow-up in 100 patients. Gastrointest Endosc 1999;49:1-7.

33. Vogel A and Venugopalan V. Mechanisms of pulsed laser ablation of biological tissues. Chemical Reviews 2003;103:2079-2079.

34. McKenzie AL. Physics of thermal processes in laser-tissue interactions. Physics in Medicine & Biology 1990;35:1175-1209.

35. Anderson RR and Parrish JA. Selective photothermolysis: precise microsurgery by selective absorption of pulsed radiation. Science 1983;220:524-527.

36. Jacques SL. Role of tissue optics and pulse duration on tissue effects during high-power laser irradiation. Applied Optics 1993;32:2447-2454.

37. Nahen K and Vogel A. Investigations on acoustic on-line monitoring of IR laser ablation of burned skin. Lasers in Surgery & Medicine 1999;25:69-78.

38. Jerath MR, Kaisig D, Rylander HG, 3rd and Welch AJ. Calibrated real-time control of lesion size based on reflectance images. Applied Optics 1993;32:1200-1209.

39. Jerath MR, Gardner CM, Rylander HG, 3rd and Welch AJ. Dynamic optical property changes: implications for reflectance feedback control of photocoagulation. Journal of Photochemistry & Photobiology B - Biology 1992;16:113-126.

40. Deckelbaum LI. Coronary laser angioplasty. Lasers in Surgery & Medicine 1994;14:101-110.

41. Kim BM, Feit MD, Rubenchik AM, Mammini BM and Da Silva LB. Optical feedback signal for ultra short laser pulse ablation of tissue. Applied Surface Science 1998; 127-129:857-862.

42. Brinkmann R, Hansen C, Mohrenstecher D, Scheu M and Birngruber R. Analysis of cavitation dynamics during pulsed laser tissue ablation by optical on-line monitoring. Selected Topics in Quantum Electronics, IEEE Journal of 1996;2:826.

43. Whelan WM, Davidson SRH, Chin LCL and Vitkin IA. A Novel Strategy For Monitoring Laser Thermal Therapy Based on Changes in Optothermal Properties of Heated Tissues. International Journal of Thermophysics 2005;26:233-241.

44. Boppart SA, Herrmann J, Pitris C, Stamper DL, Brezinski ME and Fujimoto JG. High-resolution optical coherence tomography-guided laser ablation of surgical tissue. Journal of Surgical Research 1999;82:275-284.

45. Thomsen SL, Jacques SL and Flock ST. Microscopic correlates of macroscopic optical property changes during thermal coagulation of myocardium. Proceedings of the SPIE 1990;1202:2-11.

46. Maitland DJ and Walsh JT, Jr. Quantitative measurements of linear birefringence during heating of native collagen. Lasers Surg Med 1997;20:310-318.

47. Kimel S, Svaasand LO, Hammer-Wilson M, Schell MJ, Milner TE, Nelson JS and Berns MW. Differential vascular response to laser photothermolysis. Journal of Investigative Dermatology 1994;103:693-700.

48. Khan MH, Sink RK, Manstein D, Eimerl D and Anderson RR. Intradermally focused infrared laser pulses: Thermal effects at defined tissue depths. Lasers in Surgery and Medicine 2005;36:270-280.

49. Neumann RA, Knobler RM, Pieczkowski F and Gebhart W. Enzyme histochemical analysis of cell viability after argon laser-induced coagulation necrosis of the skin. Journal of the American Academy of Dermatology 1991;25:991-998.

50. Nadkarni S, Helg T, Bouma BE, Chan RC, Minsky MS, Chau AH, Motz J, Houser SL and Tearney GJ. Characterization of atherosclerotic plaques by laser speckle analysis. Circulation 2005; 112:885-892.

51. Zimnyakov DA, Agafonov DN, Sviridov AP, Omel'chenko AI, Kuznetsova LV and Bagratashvili VN. Speckle-contrast monitoring of tissue thermal modification. Appl Opt 2002;41:5989-5996.

52. Pierce MC, Sheridan RL, Park BH, Cense B and de Boer JF. Collagen denaturation can be quantified in burned human skin using polarization-sensitive optical coherence tomography. Bums 2004;30:511-517.

53. de Boer JF, Milner TE, van Gemert MJC and Nelson JS. Two-dimensional birefringence imaging in biological tissue using polarization sensitive optical coherence tomography. Optics Letters 1997;22:934-936.

54. Morelli JG, Tan OT, Garden J, Margolis R, Seki Y, Boll J, Carney JM, Anderson RR, Furumoto H and Parrish JA. Tunable dye laser (577 nm) treatment of port wine stains. Lasers Surg Med 1986;6:94-99.

55. Chen ZP, Milner TE, Dave D and Nelson JS. Optical Doppler tomographic imaging of fluid flow velocity in highly scattering media. Optics Letters 1997;22:64-66.

56. Izatt JA, Kulkarni MD, Yazdanfar S, Barton JK and Welch AJ. In vivo bidirectional color Doppler flow imaging of picoliter blood volumes using optical coherence tomography. Optics Letters 1997;22:1439.

57. Barton JK, Welch AJ and Izatt JA. Investigating pulsed dye laser-blood vessel interaction with color Doppler optical coherence tomography. Optics Express 1998;3:251-256.

58. French PMW, Rizvi NH, Taylor JR and Shestakov AV. Continuous-wave mode-locked Cr4+:YAG laser. Optics Letters 1993;18:39-41.

59. Sennaroglu A, Pollock CR and Nathel H. Efficient continuous-wave chromium-doped YAG laser. Journal of the Optical Society of America B 1995;12:930-937.

60. Bouma B, Gouveia-Neto A, Izatt JA, Russell J, Sierra R, Keller U and Fujimoto JG. Hybrid mode locking of a flash-lamp-pumped Ti:Al2O3 laser. Optics Letters 1994;19:1858-1860.

61. Bouma B, Tearney GJ, Boppart SA, Hee MR, Brezinski ME and Fujimoto JG. High-resolution optical coherence tomographic imaging using a mode-locked Ti:Al2O3 laser source. Optics Letters 1995;20:1486-1488.

62. Bouma BE and Fujimoto JG. Compact Kerr-lens mode-locked resonators. Optics Letters 1996;21:134-136.

63. Bouma BE, Nelson LE, Tearney GJ, Jones DJ, Brezinski ME and Fujimoto JG. Optical coherence tomographic imaging of human tissue at 1.55 $\mu$m and 1.81 $\mu$m using Er and Tm-doped fiber sources. Journal of Biomedical Optics 1998;3:76-79.

64. Bouma BE, Ramaswamy-Paye M and Fujimoto JG. Compact resonator designs for mode-locked solid-state lasers. Applied Physics B (Lasers and Optics) 1997;65:213-220.

65. Bouma BE, Tearney GJ, Bilinsky IP, Golubovic B and Fujimoto JG. Self-phase-modulated Kerr-lens mode-locked Cr:forsterite laser source for optical coherence tomography. Optics Letters 1996;21:1839-1841.

The foregoing merely illustrates the principles of the invention. Various modifications and alterations to the described embodiments will be apparent to those skilled in the art in view of the teachings herein. Indeed, the arrangements, systems and methods according to the exemplary embodiments of the present invention can be used with and/or implement any OCT system, OFDI system, SD-OCT system or other imaging systems, and for example with those described in International Patent Application PCT/US2004/029148, filed September 8, 2004, U.S. Patent Application No. 11/266,779, filed November 2, 2005, and U.S. Patent Application No. 10/501,276, filed July 9, 2004.

## Claims

1. An ex-vivo process for obtaining information associated with at least one portion of a biological sample (2270), comprising:

   forwarding at least one first electro-magnetic radiation to a section near or in the at least one portion of the sample (2270);
   receiving at least one second electro-magnetic radiation provided from said section near or in the at least one portion of the biological sample (2270); and
   identifying a deformation of the section at a plurality of depths as a function of at least one of (i) a phase of said at least one second electro-magnetic radiation provided from the section, or (ii) a rate of change of at least one of the phase or an amplitude of the at least one second electro-magnetic radiation.

2. The process according to claim 1, further comprising:

   generating an interferometric signal associated with the at least one second electro-magnetic radiation; and
   determining the phase of the at least one second electro-magnetic radiation using the interferometric signal.

3. The process according to claim 2, further comprising measuring the interferometric signal as a function of a wavelength of the at least one second electro-magnetic radiation.

4. The process according to claim 1, wherein the at least one first electro-magnetic radiation has a wavelength that varies over time.

5. The process according to claim 1, wherein a temperature change in the at least one portion of the sample (2270) is caused using a laser arrangement.

6. The process according to claim 1, further comprising defining a border between at least one irreversibly changed portion and an unchanged portion of the biological sample as a function of information associated with the deformation.

7. The process according to claim 6, wherein the at least one changed portion is at least one of denatured, damaged or destroyed.

8. The process according to claim 1, further comprising:

generating an interferometric signal associated with the at least one second electro-magnetic radiation; and determining the amplitude of the at least one second electro-magnetic radiation using the interferometric signal.

9. The process according to claim 8, further comprising measuring the interferometric signal as a function of a wavelength of the at least one second electro-magnetic radiation.

10. The process according to claim 1, wherein the at least one first electro-magnetic radiation is forwarded to the section in the sample at a particular wavelength; and further comprising:

during the forwarding of the at least one first electro-magnetic radiation, controlling a temperature distribution in the sample by modifying the particular wavelength of the electro-magnetic radiation.

11. The process according to claim 1, wherein the forwarding procedure comprises applying a beam of the electro-magnetic radiation to the section, wherein a cross-sectional area of the beam is at most about 1/10$^{th}$ of an entire area of the section, and further comprising:

applying the beam to the at least one portion at least one of (i) while modulating a wavelength of the laser radiation, or (ii) while monitoring a depth of the application of the laser radiation

12. A system for obtaining information associated with at least one portion of a biological sample (2210; 2270; 1120; 1050b; 2510), comprising:

a particular arrangement (2200; 2640a, 2641a; 2800b; 1101, 1100, 1105; 1200, 1245) configured to forward at least one first electro-magnetic radiation to a section near or in the at least one portion of the biological sample; a first arrangement configured to receive at least one second electro-magnetic radiation provided which is based on the at least one first radiation from the section near or in the at least one portion of the biological sample; and a second arrangement configured to identify a deformation of the section at a plurality of depths as a function of at least one of (i) a phase of the at least one second electro-magnetic radiation provided from the section, or (ii) a rate of change of at least one of the phase or an amplitude of the at least one second electro-magnetic radiation.

13. The system according to claim 12, wherein the second arrangement is configured to define a border between at least one changed portion and an unchanged portion of the sample as a function of the information associated with the deformation.

14. The system according to claim 12, further comprising a third arrangement configured to cause a temperature change in the at least one portion of the biological sample.

**Patentansprüche**

1. Ein Ex-vivo-Prozess zum Erhalten von Informationen, die zumindest einem Teil einer biologischen Probe (2270) zugeordnet sind, wobei der Prozess folgende Schritte aufweist:

Weiterleiten zumindest einer ersten elektromagnetischen Strahlung an einen Abschnitt, der sich in der Nähe des oder in dem zumindest einen Teil der Probe (2270) befindet; Empfangen zumindest einer zweiten elektromagnetischen Strahlung, die von dem Abschnitt, der sich in der Nähe des oder in dem zumindest einen Teil der biologischen Probe (2270) befindet, bereitgestellt wird; und Identifizieren einer Verformung des Abschnitts an einer Mehrzahl von Tiefen in Abhängigkeit von zumindest entweder (i) einer Phase der zumindest einen zweiten elektromagnetischen Strahlung, die von dem Abschnitt bereitgestellt wird, und/oder (ii) einer Änderungsrate zumindest entweder der Phase und/oder einer Amplitude der zumindest einen zweiten elektromagnetischen Strahlung.

2. Der Prozess gemäß Anspruch 1, der ferner folgende Schritte aufweist:

Erzeugen eines interferometrischen Signals, das der zumindest einen zweiten elektromagnetischen Strahlung zugeordnet ist; und

Bestimmen der Phase der zumindest einen zweiten elektromagnetischen Strahlung unter Verwendung des interferometrischen Signals.

3. Der Prozess gemäß Anspruch 2, der ferner ein Messen des interferometrischen Signals in Abhängigkeit von einer Wellenlänge der zumindest einen zweiten elektromagnetischen Strahlung aufweist.

4. Der Prozess gemäß Anspruch 1, bei dem die zumindest eine erste elektromagnetische Strahlung eine Wellenlänge aufweist, die über die Zeit hinweg variiert.

5. Der Prozess gemäß Anspruch 1, bei dem eine Temperaturänderung in dem zumindest einen Teil der Probe (2270) unter Verwendung einer Laseranordnung bewirkt wird.

6. Der Prozess gemäß Anspruch 1, der ferner ein Definieren einer Grenze zwischen zumindest einem irreversibel veränderten Teil und einem unveränderten Teil der biologischen Probe in Abhängigkeit von Informationen aufweist, die der Verformung zugeordnet sind.

7. Der Prozess gemäß Anspruch 6, bei dem der zumindest eine veränderte Teil zumindest entweder denaturiert und/oder beschädigt und/oder zerstört wird.

8. Der Prozess gemäß Anspruch 1, der ferner folgende Schritte aufweist:

Erzeugen eines interferometrischen Signals, das der zumindest einen zweiten elektromagnetischen Strahlung zugeordnet ist; und

Bestimmen der Amplitude der zumindest einen zweiten elektromagnetischen Strahlung unter Verwendung des interferometrischen Signals.

9. Der Prozess gemäß Anspruch 8, der ferner ein Messen des interferometrischen Signals in Abhängigkeit von einer Wellenlänge der zumindest einen zweiten elektromagnetischen Strahlung aufweist.

10. Der Prozess gemäß Anspruch 1, bei dem die zumindest eine erste elektromagnetische Strahlung mit einer bestimmten Wellenlänge an den Abschnitt in der Probe weitergeleitet wird; und der ferner folgenden Schritt aufweist:

während des Weiterleitens der zumindest einen ersten elektromagnetischen Strahlung, Steuern einer Temperaturverteilung in der Probe durch Modifizieren der bestimmten Wellenlänge der elektromagnetischen Strahlung.

11. Der Prozess gemäß Anspruch 1, bei dem die Weiterleitungsprozedur ein Anlegen eines Strahls der elektromagnetischen Strahlung an den Abschnitt aufweist, wobei eine Querschnittsfläche des Strahls höchstens etwa 1/10 einer Gesamtfläche des Abschnitts beträgt, und der ferner folgenden Schritt aufweist:

Anlegen des Strahls an den zumindest einen Teil zumindest entweder (i) während eines Modulierens einer Wellenlänge der Laserstrahlung und/oder (ii) während eines Überwachens einer Tiefe des Anlegens der Laserstrahlung

12. Ein System zum Erhalten von Informationen, die zumindest einem Teil einer biologischen Probe (2210; 2270; 1120; 1050b; 2510) zugeordnet sind, wobei das System folgende Merkmale aufweist:

eine bestimmte Anordnung (2200; 2640a, 2641a; 2800b; 1101, 1100, 1105; 1200, 1245), die dazu konfiguriert ist, zumindest eine erste elektromagnetische Strahlung an einen Abschnitt weiterzuleiten, der sich in der Nähe des oder in dem zumindest einen Teil der biologischen Probe befindet;
eine erste Anordnung, die dazu konfiguriert ist, zumindest eine zweite bereitgestellte elektromagnetische Strahlung zu empfangen, die auf der zumindest einen ersten Strahlung von dem Abschnitt beruht, der sich in der Nähe des oder in dem zumindest einen Teil der biologischen Probe befindet; und
eine zweite Anordnung, die dazu konfiguriert ist, eine Verformung des Abschnitts an einer Mehrzahl von Tiefen in Abhängigkeit von zumindest entweder (i) einer Phase der zumindest einen zweiten elektromagnetischen Strahlung, die von dem Abschnitt bereitgestellt wird, und/oder (ii) einer Änderungsrate zumindest entweder der Phase und/oder einer Amplitude der zumindest einen zweiten elektromagnetischen Strahlung zu identifizieren.

**13.** Das System gemäß Anspruch 12, bei dem die zweite Anordnung dazu konfiguriert ist, eine Grenze zwischen zumindest einem veränderten Teil und einem unveränderten Teil der Probe in Abhängigkeit von den der Verformung zugeordneten Informationen zu definieren.

**14.** Das System gemäß Anspruch 12, das ferner eine dritte Anordnung aufweist, die dazu konfiguriert ist, in dem zumindest einen Teil der biologischen Probe eine Temperaturänderung zu bewirken.

**Revendications**

**1.** Procédé ex vivo pour l'obtention d'informations associées à au moins une partie d'un échantillon biologique (2270), comprenant :

la transmission d'au moins un premier rayonnement électromagnétique à une section proche ou à l'intérieur de l'au moins une partie de l'échantillon (2270) ;
la réception d'au moins un second rayonnement électromagnétique à partir de ladite section proche ou à l'intérieur de l'au moins une partie de l'échantillon biologique (2270) ; et
l'identification d'une déformation de la section au niveau d'une pluralité de profondeurs en tant que fonction d'au moins l'un d'entre (i) une phase dudit au moins un second rayonnement électromagnétique fourni à partir de la section, ou (ii) une vitesse de modification d'au moins l'un d'entre la phase ou une amplitude de l'au moins un second rayonnement électromagnétique.

**2.** Procédé selon la revendication 1, comprenant en outre :

la génération d'un signal interférométrique associé à l'au moins un second rayonnement électromagnétique ; et
la détermination de la phase de l'au moins un second rayonnement électromagnétique par l'utilisation du signal interférométrique.

**3.** Procédé selon la revendication 2, comprenant en outre la mesure du signal interférométrique en tant que fonction d'une longueur d'onde de l'au moins un second rayonnement électromagnétique.

**4.** Procédé selon la revendication 1, dans lequel l'au moins un premier rayonnement électromagnétique présente une longueur d'onde qui varie avec le temps.

**5.** Procédé selon la revendication 1, dans lequel une modification de la température dans l'au moins une partie de l'échantillon (2270) est provoquée par l'utilisation d'un agencement à laser.

**6.** Procédé selon la revendication 1, comprenant en outre la définition d'une limite entre au moins une partie irréversiblement modifiée et une partie non modifiée de l'échantillon biologique en tant que fonction d'informations associées à la déformation.

**7.** Procédé selon la revendication 6, dans lequel l'au moins une partie modifiée est au moins l'un d'entre dénaturée, endommagée ou détruite.

**8.** Procédé selon la revendication 1, comprenant en outre :

la génération d'un signal interférométrique associé à l'au moins un second rayonnement électromagnétique ; et
la détermination de l'amplitude de l'au moins un second rayonnement électromagnétique par l'utilisation du signal interférométrique.

**9.** Procédé selon la revendication 8, comprenant en outre la mesure du signal interférométrique en tant que fonction d'une longueur d'onde de l'au moins un second rayonnement électromagnétique.

**10.** Procédé selon la revendication 1, dans lequel l'au moins un premier rayonnement électromagnétique est transmis à la section dans l'échantillon à une longueur d'onde particulière ; et comprenant en outre :

pendant la transmission de l'au moins un premier rayonnement électromagnétique, le contrôle d'une répartition des températures dans l'échantillon par la modification de la longueur d'onde particulière du rayonnement électromagnétique.

11. Procédé selon la revendication 1, dans lequel le processus de transmission comprend l'application d'un faisceau du rayonnement électromagnétique à la section, dans lequel une superficie en coupe du faisceau est au plus d'environ 1/10$^e$ d'une superficie totale de la section, et comprenant en outre :

l'application du faisceau à l'au moins une partie au moins l'un d'entre (i) pendant la modulation d'une longueur d'onde du rayonnement laser, ou (ii) pendant la surveillance d'une profondeur de l'application du rayonnement laser.

12. Système pour l'obtention d'informations associées à au moins une partie d'un échantillon biologique (2210 ; 2270 ; 1120 ; 1050b ; 2510), comprenant :

un agencement particulier (2200 ; 2640a, 2641a ; 2800b ; 1101, 1100, 1105 ; 1200, 1245) configuré pour transmettre au moins un premier rayonnement électromagnétique à une section proche ou à l'intérieur de l'au moins une partie de l'échantillon biologique ;
un premier agencement configuré pour recevoir au moins un second rayonnement électromagnétique fourni qui est basé sur l'au moins un premier rayonnement à partir de la section proche ou à l'intérieur de l'au moins une partie de l'échantillon biologique ; et
un deuxième agencement configuré pour identifier une déformation de la section au niveau d'une pluralité de profondeurs en tant que fonction d'au moins l'un d'entre (i) une phase de l'au moins un second rayonnement électromagnétique fourni à partir de la section, ou (ii) une vitesse de modification de l'au moins l'un de la phase ou d'une amplitude de l'au moins un second rayonnement électromagnétique.

13. Système selon la revendication 12, dans lequel le deuxième agencement est configuré pour définir une limite entre au moins une partie modifiée et une partie non modifiée de l'échantillon en tant que fonction des informations associées à la déformation.

14. Système selon la revendication 12, comprenant en outre un troisième agencement configuré pour provoquer une modification de la température dans l'au moins une partie de l'échantillon biologique.

Side View

Esophagus
2050

Focused Spot
2060

Probe Scanner
2000

Baloon
2040

Imaging Beam
2030

Inner Core
2010

Transparent Sheaths
2020

F I G. 1A

2070

F I G. 1B

2100                 2110

**F I G. 2**
PRIOR ART

2150

**F I G. 3**

FIG. 4

F I G. 5

EP 1 983 920 B1

FIG. 6

2450 2460

A

Pre-laser OFDI intensity

500 µm

B

During laser OFDI intensity

500 µm

C

Pre-laser OFDI birefringence

D

During laser OFDI birefringence

2470 2480

# FIG. 7

$\phi = 0$      $\phi = 360$

5.6 cm

proximal

4.5 cm

distal

2490

# FIG. 8

FIG. 9

2590

2595

FIG. 10

FIG. 11

F I G. 12

F I G. 13

F I G. 14

F I G. 15

F I G. 16

F I G. 17

F I G. 18

EP 1 983 920 B1

**F I G. 19**

Imeging system
400

405a    405b    410

Rotary
Coupler/
Pullback

405c

Catheter
415

**F I G. 20**

Imaging
system
300

305

Rotary
Coupler/
Pullback

310

WDM
330

Splitter
335

331    332    336a    336b

rigid
section
315

flexible
section
320

Catheter
325

therapy beam
1120

imaging beam
1125

fiber
1101

GRIN Lens
1100

WDM
Prism
1105

GRIN
Lens
1110

Prism
1115

F I G. 21

## Side View

## End View

F I G. 22

EP 1 983 920 B1

**F I G. 23**

baloon
1255

fiber (therapy light)
1200

focusing optics
(therapy beam)
1250

prism
1245

Outer Sheath
1240

Inner Sheath
1235

motor elec. connection
1205

Motor
1260

Optical Core
1230

fiber (imaging light)
1210

focusing optics
(imaging beam)
1215

prism
1220

Reflector
1225

**F I G. 24**

600

| Low-Power Wavelength Tunable Source |

605

| Broadband Booster Amplifier |

500a     500b     500c     500d

| LD(x,$\lambda_1$) | LD(y,$\lambda_1$) | LD(x,$\lambda_2$) | LD(y,$\lambda_2$) |

Pol Mux    505a        Pol Mux    505b

510   $\lambda$ Mux

Mode-Scambler 525 (optional)

Single Mode Fiber 515

Multi-Mode Fiber 520 (optional)

F I G. 25

Laser Diode Bar 700

701a
701b
701c
701d
701e
701f
701g

Grating 710

Focusing Optics 705

Partially Reflective Mirror 715

Laser Output 720

F I G. 26

Treatment Light
2500

Galvo Mirror
2520

Imaging Light
2570

Collimated Beam
(1.1 mm diameter)
2510

Collimated Beam
(4.3 mm diameter)
2580

2530

Coverslip 2540

Tissue 2550

Backing 2560

FIG. 27

FIG. 28

START → Temperature change can be caused in a portion of the sample → A first electro-magnetic radiation forwarded to section near or in portion of sample → Deformation of section can be identified at a plurality of depths as a function of phase of a second electro-magnetic radiation provided from section, and/or rate of change of phase and/or an amplitude of second electro-magnetic radiation → STOP

3100    3110    3120

**F I G. 29A**

START → Electro-magnetic radiation is provided to section in sample at particular wavelength → Temperature distribution is controlled by modifying particular wavelength of electro-magnetic radiation when electro-magnetic radiation is provided to section → STOP

3130    3140

**F I G. 29B**

START → Beam of laser radiation is provided to the portion, whereas a cross-sectional area of beam is at most about 1/10th of entire area of the portion → Beam is applied to portion based on predetermined pattern, while modulating wavelength of laser radiation, and/or while monitoring depth of application of laser radiation → STOP

3150    3160

**F I G. 29C**

EP 1 983 920 B1

**EP 1 983 920 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9628212 A
- EP 0933096 A2
- US 2004029148 W
- US 50127604 A
- US 26677905 A

**Non-patent literature cited in the description**

- *Cancer,* 1998, vol. 83, 2049-2053
- **BARR H ; STONE N ; REMBACKEN B.** Endoscopic therapy for Barrett's oesophagus. *Gut,* 2005, vol. 54, 875-884
- **JOHNSTON MH.** Technology insight: ablative techniques for Barrett's esophagus - current and emerging trends. *Nature Clinical Practice Gastroenterology & Hepatology,* 2005, vol. 2, 323-330
- **FALK GW ; CHITTAJALLU R ; GOLDBLUM JR ; BISCOTTI CV ; GEISINGER KR ; PETRAS RE ; BIRGISSON S ; RICE TW ; RICHTER JE.** Surveillance of patients with Barrett's esophagus for dysplasia and cancer with balloon cytology. *Gastroenterology,* 1997, vol. 112, 1787-1797
- **SPECHLER SJ.** Barrett's esophagus: should we brush off this ballooning problem?. *Gastroenterology,* 1997, vol. 112, 2138-2142
- **KUBBA AK ; POOLE NA ; WATSON A.** Role of p53 assessment in management of Barrett's esophagus. *Dig Dis Sci,* 1999, vol. 44, 659-667
- **REID BJ.** p53 and neoplastic progression in Barrett's esophagus. *Am J Gastroenterol,* 2001, vol. 96, 1321-1323
- **SHARMA P ; WESTON AP ; TOPALOVSKI M ; CHERIAN R ; BHATTACHARYYA A ; SAMPLINER RE.** Magnification chromoendoscopy for the detection of intestinal metaplasia and dysplasia in Barrett's oesophagus. *GUT,* 2003, vol. 52, 24-27
- **KUIPERS EJ ; HARINGSMA J.** Diagnostic and therapeutic endoscopy. *Journal of Surgical Oncology,* 2005, vol. 92, 203-209
- **GEORGAKOUDI I ; JACOBSON BC ; VAN DAM J ; BACKMAN V ; WALLACE MB ; MULLER MG ; ZHANG Q ; BADIZADEGAN K ; SUN D ; THOMAS GA.** Fluorescence, reflectance, and light-scattering spectroscopy for evaluating dysplasia in patients with Barrett's esophagus. *Gastroenterology,* 2001, vol. 120, 1620-1629
- **ADRAIN AL ; TER HC ; CASSIDY MJ ; SCHIANO TD ; LIU JB ; MILLER LS.** High-resolution endoluminal sonography is a sensitive modality for the identification of Barrett's metaplasia. *Gastrointest Endosc,* 1997, vol. 46, 147-151
- **CANTO MI.** Vital staining and Barrett's esophagus. *Gastrointest Endosc,* 1999, vol. 49, 12-16
- **HUANG D ; SWANSON EA ; LIN CP ; SCHUMAN JS ; STINSON WG ; CHANG W ; HEE MR ; FLOTTE T ; GREGORY K ; PULIAFITO CA.** Optical coherence tomography. *Science,* 1991, vol. 254, 1178-1181
- **TEARNEY GJ ; BREZINSKI ME ; BOUMA BE ; BOPPART SA ; PITVIS C ; SOUTHERN JF ; FUJIMOTO JG.** In vivo endoscopic optical biopsy with optical coherence tomography. *Science,* 1997, vol. 276, 2037-2039
- **EVANS JA ; PONEROS JM ; BOUMA BE ; BRESSNER J ; HALPERN EF ; SHISHKOV M ; LAUWERS GY ; MINO-KENUDSON M ; NISHIOKA NS ; TEARNEY GJ.** Optical Coherence Tomography to Identify Intramucosal Carcinoma and High Grade Dysplasia in Barrett's Esophagus. *Clinical Gastroenterology and Hepatology,* 2005, vol. 4, 38-43
- **PONEROS JM ; BRAND S ; BOUMA BE ; TEARNEY GJ ; COMPTON CC ; NISHIOKA NS.** Diagnosis of Specialized Intestinal Metaplasia by Optical Coherence Tomography. *Gastroenterology,* 2001, vol. 120, 7-12
- **BRAND S ; PONEROS JM ; BOUMA BE ; TEARNEY GJ ; COMPTON CC ; NISHIOKA NS.** Optical Coherent Tomography in the Gastrointestinal Tract. *Endoscopy,* 2000, vol. 32, 796-803
- **DE BOER JF ; CENSE B ; PARK BH ; PIERCE MC ; TEARNEY GJ ; BOUMA BE.** Improved signal-to-noise ratio in spectral-domain compared with time-domain optical coherence tomography. *Optics Letters,* 2003, vol. 28, 2067-2069
- **CHOMA MA ; SARUNIC MV ; CHANGHUEI Y ; IZATT JA.** Sensitivity advantage of swept source and Fourier domain optical coherence tomography. *Optics Express,* 2003, vol. 11, 2183-2189

- **LEITGEB R ; HITZENBERGER CK ; FERCHER AF.** Performance of Fourier domain vs. time domain optical coherence tomography. *Optics Express,* 2003, vol. 11, 889-894
- **YUN SH ; TEARNEY GJ ; DE BOER JF ; IFTIMIA N ; BOUMA BE.** High-speed optical frequency-domain imaging. *Optics Express,* 2003, vol. 11, 2953-2963
- **YUN SH ; BOUDOUX C ; TEARNEY GJ ; BOUMA BE.** High-speed wavelength-swept semiconductor laser with a polygon-scanner-based wavelength filter. *Optics Letters,* 2003, vol. 28, 1981-1983
- **OH WY ; YUN SH ; TEARNEY GJ ; BOUMA BE.** 115 kHz tuning repetition rate ultrahigh-speed wavelength-swept semiconductor laser. *Optics Letters,* 2005, vol. 30, 3159-3161
- **VAKOC BJ ; YUN SH ; DE BOER JF ; TEARNEY GJ ; BOUMA BE.** Phase-resolved optical frequency domain imaging. *Optics Express,* 2005, vol. 13, 5483-5493
- **BROWN SB ; BROWN EA ; WALKER I.** The present and future role of photodynamic therapy in cancer treatment. *Lancet Oncol,* 2004, vol. 5, 497-508
- **VAN DEN BOOGERT J ; VAN HILLEGERSBERG R ; SIERSEMA PD ; DE BRUIN RW ; TILANUS HW.** Endoscopic ablation therapy for Barrett's esophagus with high-grade dysplasia: a review. *Am J Gastroenterol,* 1999, vol. 94, 1153-1160
- **SAMPLINER RE ; FENNERTY B ; GAREWAL HS.** Reversal of Barrett's esophagus with acid suppression and multipolar electrocoagulation: preliminary results. *Gastrointest Endosc,* 1996, vol. 44, 532-535
- **SAMPLINER RE.** Endoscopic ablative therapy for Barrett's esophagus: current status. *Gastrointest Endosc,* 2004, vol. 59, 66-69
- **SOETIKNO RM ; GOTODA T ; NAKANISHI Y ; SOEHENDRA N.** Endoscopic mucosal resection. *Gastrointest Endosc,* 2003, vol. 57, 567-579
- **GANZ RA ; UTLEY DS ; STERN RA ; JACKSON J ; BATTS KP ; TERMIN P.** Complete ablation of esophageal epithelium with a balloon-based bipolar electrode: a phased evaluation in the porcine and in the human esophagus. *Gastrointest Endosc,* 2004, vol. 60, 1002-1010
- **MARK H. JOHNSTON ; BROOKS D. CASH ; CATHY A. DYKES ; HALISHA S. MAYS ; LAVONNE R. JOHNSTON.** Cryoablation of Dysplasia in Barrett's Esophagus (BE) and Early Stage Esophageal Cancer. *Gastrointest Endosc,* 2006, vol. 63, AB223
- **OVERHOLT BF ; PANJEHPOUR M ; HAYDEK JM.** Photodynamic therapy for Barrett's esophagus: follow-up in 100 patients. *Gastrointest Endosc,* 1999, vol. 49, 1-7
- **VOGEL A ; VENUGOPALAN V.** Mechanisms of pulsed laser ablation of biological tissues. *Chemical Reviews,* 2003, vol. 103, 2079-2079
- **MCKENZIE AL.** Physics of thermal processes in laser-tissue interactions. *Physics in Medicine & Biology,* 1990, vol. 35, 1175-1209
- **ANDERSON RR ; PARRISH JA.** Selective photothermolysis: precise microsurgery by selective absorption of pulsed radiation. *Science,* 1983, vol. 220, 524-527
- **JACQUES SL.** Role of tissue optics and pulse duration on tissue effects during high-power laser irradiation. *Applied Optics,* 1993, vol. 32, 2447-2454
- **NAHEN K ; VOGEL A.** Investigations on acoustic on-line monitoring of IR laser ablation of burned skin. *Lasers in Surgery & Medicine,* 1999, vol. 25, 69-78
- **JERATH MR ; KAISIG D ; RYLANDER HG, 3RD ; WELCH AJ. CALIBRATED.** real-time control of lesion size based on reflectance images. *Applied Optics,* 1993, vol. 32, 1200-1209
- **JERATH MR ; GARDNER CM ; RYLANDER HG, 3RD ; WELCH AJ.** Dynamic optical property changes: implications for reflectance feedback control of photocoagulation. *Journal of Photochemistry & Photobiology B - Biology,* 1992, vol. 16, 113-126
- **DECKELBAUM LI.** Coronary laser angioplasty. *Lasers in Surgery & Medicine,* 1994, vol. 14, 101-110
- **KIM BM ; FEIT MD ; RUBENCHIK AM ; MAMMINI BM ; DA SILVA LB.** Optical feedback signal for ultra short laser pulse ablation of tissue. *Applied Surface Science,* 1998, vol. 127-129, 857-862
- *Selected Topics in Quantum Electronics, IEEE Journal,* 1996, vol. 2, 826
- **WHELAN WM ; DAVIDSON SRH ; CHIN LCL ; VITKIN IA.** A Novel Strategy For Monitoring Laser Thermal Therapy Based on Changes in Optothermal Properties of Heated Tissues. *International Journal of Thermophysics,* 2005, vol. 26, 233-241
- **BOPPART SA ; HERRMANN J ; PITRIS C ; STAMPER DL ; BREZINSKI ME ; FUJIMOTO JG.** High-resolution optical coherence tomography-guided laser ablation of surgical tissue. *Journal of Surgical Research,* 1999, vol. 82, 275-284
- **THOMSEN SL ; JACQUES SL ; FLOCK ST.** Microscopic correlates of macroscopic optical property changes during thermal coagulation of myocardium. *Proceedings of the SPIE,* 1990, vol. 1202, 2-11
- **MAITLAND DJ ; WALSH JT.** Jr. Quantitative measurements of linear birefringence during heating of native collagen. *Lasers Surg Med,* 1997, vol. 20, 310-318
- **KIMEL S ; SVAASAND LO ; HAMMER-WILSON M ; SCHELL MJ ; MILNER TE ; NELSON JS ; BERNS MW.** Differential vascular response to laser photothermolysis. *Journal of Investigative Dermatology,* 1994, vol. 103, 693-700

- **KHAN MH ; SINK RK ; MANSTEIN D ; EIMERL D ; ANDERSON RR.** Intradermally focused infrared laser pulses: Thermal effects at defined tissue depths. *Lasers in Surgery and Medicine,* 2005, vol. 36, 270-280
- **NEUMANN RA ; KNOBLER RM ; PIECZKOWSKI F ; GEBHART W.** Enzyme histochemical analysis of cell viability after argon laser-induced coagulation necrosis of the skin. *Journal of the American Academy of Dermatology,* 1991, vol. 25, 991-998
- **NADKARNI S ; HELG T ; BOUMA BE ; CHAN RC ; MINSKY MS ; CHAU AH ; MOTZ J ; HOUSER SL ; TEARNEY GJ.** Characterization of atherosclerotic plaques by laser speckle analysis. *Circulation,* 2005, vol. 112, 885-892
- **ZIMNYAKOV DA ; AGAFONOV DN ; SVIRIDOV AP ; OMEL'CHENKO AI ; KUZNETSOVA LV ; BAGRATASHVILI VN.** Speckle-contrast monitoring of tissue thermal modification. *Appl Opt,* 2002, vol. 41, 5989-5996
- **PIERCE MC ; SHERIDAN RL ; PARK BH ; CENSE B ; DE BOER JF.** Collagen denaturation can be quantified in burned human skin using polarization-sensitive optical coherence tomography. *Bums,* 2004, vol. 30, 511-517
- **DE BOER JF ; MILNER TE ; VAN GEMERT MJC ; NELSON JS.** Two-dimensional birefringence imaging in biological tissue using polarization sensitive optical coherence tomography. *Optics Letters,* 1997, vol. 22, 934-936
- **MORELLI JG ; TAN OT ; GARDEN J ; MARGOLIS R ; SEKI Y ; BOLL J ; CARNEY JM ; ANDERSON RR ; FURUMOTO H ; PARRISH JA.** Tunable dye laser (577 nm) treatment of port wine stains. *Lasers Surg Med,* 1986, vol. 6, 94-99
- **CHEN ZP ; MILNER TE ; DAVE D ; NELSON JS.** Optical Doppler tomographic imaging of fluid flow velocity in highly scattering media. *Optics Letters,* 1997, vol. 22, 64-66
- **IZATT JA ; KULKARNI MD ; YAZDANFAR S ; BARTON JK ; WELCH AJ.** In vivo bidirectional color Doppler flow imaging of picoliter blood volumes using optical coherence tomography. *Optics Letters,* 1997, vol. 22, 1439
- **BARTON JK ; WELCH AJ ; IZATT JA.** Investigating pulsed dye laser-blood vessel interaction with color Doppler optical coherence tomography. *Optics Express,* 1998, vol. 3, 251-256
- **FRENCH PMW ; RIZVI NH ; TAYLOR JR ; SHESTAKOV AV.** Continuous-wave mode-locked Cr4+:YAG laser. *Optics Letters,* 1993, vol. 18, 39-41
- **SENNAROGLU A ; POLLOCK CR ; NATHEL H.** Efficient continuous-wave chromium-doped YAG laser. *Journal of the Optical Society of America B,* 1995, vol. 12, 930-937
- **BOUMA B ; GOUVEIA-NETO A ; IZATT JA ; RUSSELL J ; SIERRA R ; KELLER U ; FUJIMOTO JG.** Hybrid mode locking of a flash-lamp-pumped Ti:Al2O3 laser. *Optics Letters,* 1994, vol. 19, 1858-1860
- **BOUMA B ; TEARNEY GJ ; BOPPART SA ; HEE MR ; BREZINSKI ME ; FUJIMOTO JG.** High-resolution optical coherence tomographic imaging using a mode-locked Ti:Al2O3 laser source. *Optics Letters,* 1995, vol. 20, 1486-1488
- **BOUMA BE ; FUJIMOTO JG.** Compact Kerr-lens mode-locked resonators. *Optics Letters,* 1996, vol. 21, 134-136
- **BOUMA BE ; NELSON LE ; TEARNEY GJ ; JONES DJ ; BREZINSKI ME ; FUJIMOTO JG.** Optical coherence tomographic imaging of human tissue at 1.55 $\mu$m and 1.81 $\mu$m using Er and Tm-doped fiber sources. *Journal of Biomedical Optics,* 1998, vol. 3, 76-79
- **BOUMA BE.** Ramaswamy-Paye M and Fujimoto JG. Compact resonator designs for mode-locked solid-state lasers. *Applied Physics B (Lasers and Optics),* 1997, vol. 65, 213-220
- **BOUMA BE ; TEARNEY GJ ; BILINSKY IP ; GOLUBOVIC B ; FUJIMOTO JG.** Self-phase-modulated Kerr-lens mode-locked Cr:forsterite laser source for optical coherence tomography. *Optics Letters,* 1996, vol. 21, 1839-1841